# EUROPEAN PATENT SPECIFICATION

(11) **EP 1 817 418 B1**
(45) Date of publication and mention of the grant of the patent: **04.06.2014**
(21) Application number: 05810979.4
(22) Date of filing: 28.11.2005
(51) Int. Cl.: C12P 21/02

(54) **PRODUCTION OF PROTEINS**
HERSTELLUNG VON PROTEINEN
PRODUCTION DE PROTEINES

(30) Priority: 30.11.2004 IL 16548404
(43) Date of publication of application: 15.08.2007
(73) Proprietor: Merck Serono SA, 1267 Coinsins, Vaud (CH)
(72) Inventor: ASSARAF, Yehuda, D. N Misgav 20175 (IL); ROTHEM, Lilah, D.N Hof Hakarmel 30885 (IL)
(74) Representative: Grünecker, Kinkeldey, Stockmair & Schwanhäusser
(86) International application number: PCT/IL2005/001263
(87) International publication number: WO 2006/059323

(56) References cited:
- WO-A-2004/078783
- US-A- 5 723 292
- US-A- 5 871 957
- SCHÜPERELI ET AL: "Efficient expression of Escherichia coli galactokinase gene in mammalian cells" PROCEEDINGS OF THE NATIONAL ACADEMY OF SCIENCES OF USA, NATIONAL ACADEMY OF SCIENCE, WASHINGTON, DC, US, vol. 79, January 1982 (1982-01), pages 257-261, XP002129147 ISSN: 0027-8424
- RYMOND B C ET AL: "THE EXPRESSION IN YEAST OF THE ESCHERICHIA-COLI GAL-K GENE ON CYC-1 GAL-K FUSION PLASMIDS" GENE (AMSTERDAM), vol. 25, no. 2-3, 1983, pages 249-262, XP009063151 ISSN: 0378-1119
- AI Y ET AL: "COMPARISON OF THE ENZYMATIC ACTIVITIES OF HUMAN GALACTOKINASE GALK1 AND A RELATED HUMAN GALACTOKINASE PROTEIN GK2" BIOCHEMICAL AND BIOPHYSICAL RESEARCH COMMUNICATIONS, ACADEMIC PRESS INC. ORLANDO, FL, US, vol. 212, no. 2, 17 July 1995 (1995-07-17), pages 687-691, XP000826357 ISSN: 0006-291X

### SEQUENCE LISTING

<110> Applied Research systems ARS Holding N.V ASSARAF, Yehuda ROTEM, Lilah
<120> Production of proteins
<130> 1024W0
<150> 165484
   <151> 2004-11-30
<160> 4
<170> PatentIn version 3.1
<210> 1
   <211> 591
   <212> PRT
   <213> Homo sapiens
<400> 1
   Met Val Pro Ser Ser Pro Ala Val Glu Lys Gln Val Pro Val Glu Pro
   1               5                   10                  15
   Gly Pro Asp Pro Glu Leu Arg Ser Trp Arg Arg Leu Val Cys Tyr Leu
               20                  25                  30
   Cys Phe Tyr Gly Phe Met Ala Gln Ile Arg Pro Gly Glu Ser Phe Ile
           35                  40                  45
   Thr Pro Tyr Leu Leu Gly Pro Asp Lys Asn Phe Thr Arg Asp Glu Val
       50                  55                  60
   Thr Asn Glu Ile Thr Pro Val Leu Ser Tyr Ser Tyr Leu Ala Val Leu
   65                  70                  75                  80
   Val Pro Val Phe Leu Leu Thr Asp Tyr Leu Arg Tyr Thr Pro Val Leu
                   85                  90                  95
   Leu Leu Gln Gly Leu Ser Phe Val Ser Val Trp Leu Leu Leu Leu Leu
               100                 105                 110
   Gly His Ser Val Ala His Met Gln Leu Met Glu Leu Phe Tyr Ser Val
           115                 120                 125
   Thr Met Ala Ala Arg Ile Ala Tyr Ser Ser Tyr Ile Phe Ser Leu Val
       130                 135                 140
   Arg Pro Ala Arg Tyr Gln Arg Val Ala Gly Tyr Ser Arg Ala Ala Val
   145                 150                 155                 160
   Leu Leu Gly Val Phe Thr Ser Ser Val Leu Gly Gln Leu Leu Val Thr
                   165                 170                 175
   Val Gly Arg Val Ser Phe Ser Thr Leu Asn Tyr Ile Ser Leu Ala Phe
               180                 185                 190
   Leu Thr Phe Ser Val Val Leu Ala Leu Phe Leu Lys Arg Pro Lys Arg
           195                 200                 205
   Ser Leu Phe Phe Asn Arg Asp Asp Arg Gly Arg Cys Glu Thr Ser Ala
       210                 215                 220
   Ser Glu Leu Glu Arg Met Asn Pro Gly Pro Gly Gly Lys Leu Gly His
   225                 230                 235                 240
   Ala Leu Arg Val Ala Cys Gly Asp Ser Val Leu Ala Arg Met Leu Arg
                   245                 250                 255
   Glu Leu Gly Asp Ser Leu Arg Arg Pro Gln Leu Arg Leu Trp Ser Leu
               260                 265                 270
   Trp Trp Val Phe Asn Ser Ala Gly Tyr Tyr Leu Val Val Tyr Tyr Val
           275                 280                 285
   His Ile Leu Trp Asn Glu Val Asp Pro Thr Thr Asn Ser Ala Arg Val
       290                 295                 300
   Tyr Asn Gly Ala Ala Asp Ala Ala Ser Thr Leu Leu Gly Ala Ile Thr
   305                 310                 315                 320
   Ser Phe Ala Ala Gly Phe Val Lys Ile Arg Trp Ala Arg Trp Ser Lys
                   325                 330                 335
   Leu Leu Ile Ala Gly Val Thr Ala Thr Gln Ala Gly Leu Val Phe Leu
               340                 345                 350
   Leu Ala His Thr Arg His Pro Ser Ser Ile Trp Leu Cys Tyr Ala Ala
           355                 360                 365
   Phe Val Leu Phe Arg Gly Ser Tyr Gln Phe Leu Val Pro Ile Ala Thr
       370                 375                 380
   Phe Gln Ile Ala Ser Ser Leu Ser Lys Glu Leu Cys Ala Leu Val Phe
   385                 390                 395                 400
   Gly Val Asn Thr Phe Phe Ala Thr Ile Val Lys Thr Ile Ile Thr Phe
                   405                 410                 415
   Ile Val Ser Asp Val Arg Gly Leu Gly Leu Pro Val Arg Lys Gln Phe
               420                 425                 430
   Gln Leu Tyr Ser Val Tyr Phe Leu Ile Leu Ser Ile Ile Tyr Phe Leu
           435                 440                 445
   Gly Ala Met Leu Asp Gly Leu Arg Asp Cys Gln Arg Gly His His Pro
       450                 455                 460
   Arg Gln Pro Pro Ala Gln Gly Leu Arg Ser Ala Ala Glu Glu Lys Ala
   465                 470                 475                 480
   Ala Gln Arg Leu Ser Val Gln Asp Lys Gly Leu Gly Gly Leu Gln Pro
                   485                 490                 495
   Ala Gln Ser Pro Pro Leu Ser Pro Glu Asp Ser Leu Gly Ala Val Gly
               500                 505                 510
   Pro Ala Ser Leu Glu Gln Arg Gln Ser Asp Pro Tyr Leu Ala Gln Ala
           515                 520                 525
   Pro Ala Pro Gln Ala Ala Glu Phe Leu Ser Pro Val Thr Thr Pro Ser
       530                 535                 540
   Pro Cys Thr Leu Ser Ser Ala Gln Ala Ser Gly Pro Glu Ala Ala Asp
   545                 550                 555                 560
   Glu Thr Cys Pro Gln Leu Ala Val His Pro Pro Gly Val Ser Lys Leu
                   565                 570                 575
   Gly Leu Gln Cys Leu Pro Ser Asp Gly Val Gln Asn Val Asn Gln
               580                 585                 590
<210> 2
   <211> 392
   <212> PRT
   <213> Homo sapiens
<400> 2
<210> 3
   <211> 1361
   <212> DNA
   <213> Homo sapiens
<400> 3
   cccgagcatc ccgcgccgac ggggctgtgc cggagcagct gtgcagagct gcaggcgcgc      60
   gtcatggctg ctttgagaca gccccaggtc gcggagctgc tggccgaggc ccggcgagcc     120
   ttccgggagg agttcggggc cgagcccgag ctggccgtgt cagcgccggg ccgcgtcaac     180
   ctcatcgggg aacacacgga ctacaaccag ggcctggtgc tgcctatggc tctggagctc     240
   atgacggtgc tggtgggcag cccccgcaag gatgggctgg tgtctctcct caccacctct     300
   gagggtgccg atgagcccca gcggctgcag tttccactgc ccacagccca gcgctcgctg     360
   gagcctggga ctcctcggtg ggccaactat gtcaagggag tgattcagta ctacccagct     420
   gcccccctcc ctggcttcag tgcagtggtg gtcagctcag tgcccctggg gggtggcctg     480
   tccagctcag catccttgga agtggccacg tacaccttcc tccagcagct ctgtccagac     540
   tcgggcacaa tagctgcccg cgcccaggtg tgtcagcagg ccgagcacag cttcgcaggg     600
   atgccctgtg gcatcatgga ccagttcatc tcacttatgg gacagaaagg ccacgcgctg     660
   ctcattgact gcaggtcctt ggagaccagc ctggtgccac tctcggaccc caagctggcc     720
   gtgctcatca ccaactctaa tgtccgccac tccctggcct ccagcgagta ccctgtgcgg 780
   cggcgccaat gtgaagaagt ggcccgggcg ctgggcaagg aaagcctccg ggaggtacaa 840
   ctggaagagc tagaggctgc cagggacctg gtgagcaaag agggcttccg gcgggcccgg 900
   cacgtggtgg gggagattcg gcgcacggcc caggcagcgg ccgccctgag acgtggcgac 960
   tacagagcct ttggccgcct catggtggag agccaccgct cactcagaga cgactatgag 1020
   gtgagctgcc cagagctgga ccagctggtg gaggctgcgc ttgctgtgcc tggggtttat 1080
   ggcagccgca tgacgggcgg tggcttcggt ggctgcacgg tgacactgct ggaggcctcc 1140
   gctgctcccc acgccatgcg gcacatccag gagcactacg gcgggactgc caccttctac 1200
   ctctctcaag cagccgatgg agccaaggtg ctgtgcttgt gaggcacccc caggacagca 1260
   cacggtgagg gtgcggggcc tgcaggccag tcccacggct ctgtgcccgg tgccatcttc 1320
   catatccggg tgctcaataa acttgtgcct ccaatgtggt a 1361
<210> 4
   <211> 1776
   <212> DNA
   <213> Homo sapiens
<400> 4
   atggtgccct ccagcccagc ggtggagaag caggtgcccg tggaacctgg gcctgacccc 60
   gagctccggt cctggcggcg cctcgtgtgc tacctttgct tctacggctt catggcgcag 120
   atacggccag gggagagctt catcaccccc tacctcctgg ggcccgacaa gaacttcacg 180
   cgggacgagg tcacgaacga gatcacgccg gtgctgtcgt actcctacct ggccgtgctg 240
   gtgcccgtgt tcctgctcac cgactacctg cgctacacgc cggtgctgct gctgcagggg 300
   ctcagcttcg tgtcggtgtg gctgctgctg ctgctgggcc actcggtggc gcacatgcag 360
   ctcatggagc tcttctacag cgtcaccatg gccgcgcgca tcgcctattc ctcctacatc 420
   ttctctctcg tgcggcccgc gcgctaccag cgtgtggccg gctactcgcg cgctgcggtg 480
   ctgctgggcg tgttcaccag ctccgtgctg ggccagctgc tggtcactgt gggccgagtc 540
   tccttctcca cgctcaacta catctcgctg gccttcctca ccttcagcgt ggtcctcgcc 600
   ctcttcctga agcgccccaa gcgcagcctc ttcttcaacc gcgacgaccg ggggcggtgc 660
   gaaacctcgg cttcggagct ggagcgcatg aatcctggcc caggcgggaa gctgggacac 720
   gccctgcggg tggcctgtgg ggactcagtg ctggcgcgga tgctgcggga gctgggggac 780
   agcctgcggc ggccgcagct gcgcctgtgg tccctctggt gggtcttcaa ctcggccggc 840
   tactacctgg tggtctacta cgtgcacatc ctgtggaacg aggtggaccc caccaccaac 900
   agtgcgcggg tctacaacgg cgcggcagat gctgcctcca cgctgctggg cgccatcacg 960
   tccttcgccg cgggcttcgt gaagatccgc tgggcgcgct ggtccaagct gctcatcgcg 1020
   ggcgtcacgg ccacgcaggc ggggctggtc ttccttctgg cgcacacgcg ccacccgagc   1080
   agcatctggc tgtgctatgc ggccttcgtg ctgttccgcg gctcctacca gttcctcgtg   1140
   cccatcgcca cctttcagat tgcatcttct ctgtctaaag agctctgtgc cctggtcttc   1200
   ggggtcaaca cgttctttgc caccatcgtc aagaccatca tcactttcat tgtctcggac   1260
   gtgcggggcc tgggcctccc ggtccgcaag cagttccagt tatactccgt gtacttcctg   1320
   atcctgtcca tcatctactt cttgggggcc atgctggatg gcctgcgcga ctgccagcgg   1380
   ggccaccacc cgcggcagcc cccggcccag ggcctgagga gtgccgcgga ggagaaggca   1440
   gcacagcgac tgagcgtgca ggacaagggc ctcggaggcc tgcagccagc ccagagcccg   1500
   ccgctttccc cagaagacag cctgggggct gtggggccag cctccctgga gcagagacag   1560
   agcgacccat acctggccca ggccccggcc ccgcaggcag ctgaattcct gagcccagtg   1620
   acaacccctt ccccctgcac tctgtcgtcc gcccaagcct caggccctga ggctgcagat  1680
   gagacttgtc cccagctggc tgtccatcct cctggtgtca gcaagctggg tttgcagtgt   1740
   cttccaagcg acggtgttca gaatgtgaac cagtga                             1776

## Description

### FIELD OF THE INVENTION

The present invention relates to a method of producing proteins in mammalian cells using permanent selection in the absence of cytotoxic drugs and, more particularly, to the production of highly pure proteins suitable for therapeutic applications.

### BACKGROUND OF THE INVENTION

Large quantities of pure proteins are needed for therapeutic as well as basic science properties. For example, hormones and growth factors (e.g., insulin, growth hormone) are in common use worldwide. On the other hand, large quantities of pure proteins are needed for protein crystallography and determination of three-dimensional structure. Advances in the field of molecular biology enable the production of large quantities of proteins by over-expressing polynucleotides encoding the protein-of-interest in host cells such as in bacterial, yeasts, fungi, insect, plant and mammalian cells.

Prokaryotic host cells offer robust production yields using inexpensive resources. However, expression of eukaryotic proteins, and especially human proteins, in prokaryotic expression systems is limited by the lack of post-translational modifications (e.g., glycosylation, carboxylation, or hydroxylation) and/or proper folding of the expressed proteins, leading to accumulation of the recombinant proteins in insoluble inclusion bodies.

Yeast expression systems offer certain advantages over prokaryotic systems since they include powerful secretory pathways and are capable of performing some limited post-translational modifications. However, expression in yeast systems typically leads to improper folding of disulphide-linked proteins.

Mammalian expression systems provide correct protein folding and appropriate post-translational modifications. However, expression in mammalian cells often results in low protein yields. To increase protein yield, stable transfection of the gene-of-interest (GOI) is performed in the presence of a cytotoxic drug [e.g., the folic acid antagonist methotrexate (MTX)] and an expression cassette including a gene circumventing the effect of the cytotoxic drug [e.g., dihydrofolate reductase (DHFR)]. However, due to the large quantities of cytotoxic drugs used during transfection and expression, proteins produced under these conditions are often contaminated with traces of drugs and are therefore unsuitable for therapeutic use.

Thus, there is a need to develop a method of producing proteins for pharmaceutical applications devoid of the above limitations.

Folic acid and reduced folates are essential vitamins which enter the mammalian cell via the human reduced folate carrier (hRFC) gene. Similarly, folic acid antagonists such as methotrexate (MTX), a potent inhibitor of DHRF, also enter mammalian cells via the RFC protein. Exposure of cells to MTX results in folate-deficiency and eventually cell death. However, previous studies demonstrated that exposure to MTX may lead to RFC inactivation which results in stable MTX-transport deficient phenotypes. In addition, inactivating mutations in the hRFC coding region, as well as silencing of the RFC promoter (primarily due to loss of expression and/or function of several transcription factors) and/or hRFC promoter methylation abolished RFC transport activity and resulted in antifolate drug resistance (Jansen et al., 1998; Drori et al., 2000a; Rothem et al., 2002; Rothem et al., 2003; Rothem et al., 2004 a, b; Worm et al., 2001). In addition, other studies, demonstrated that a gradual deprivation of leucovorin (a reduced folate) from a folic acid-free growth medium leads to marked gene amplification of the hRFC gene with a consequent 100-fold overproduction of MTX transport activity (Jansen et al., 1998; Drori et al., 2000a).

D-galactose is an essential hexose which is converted to glucose-6-phosphate in a three-step enzymatic reaction involving galactokinase (GalK1), UDP-Glucose-α-D-galactose-1-phosphate uridylyltransferase and phosphoglucomutase. Recent studies demonstrated that lack of fully functional GalK1 enzyme causes galactosemia (Novelli and Reichardt, 2000; Timson and Reece, 2003). In addition, it was found that Galactokinase-deficient hamster cells fail to grow in a medium containing D-galactose as the sole hexose source and require a medium containing D-glucose for growth (Schumperli et al., 1982).

US 5,723,292 A and US 5,871,957 A refer to methods for producing protein products in host cells and for selecting transformed cells comprising the step of transforming the host cell with a DNA molecule comprising a gene which complements a deficiency in the host cell. The host cell is a strain having a deficiency in a function necessary for normal cell growth. The gene in the DNA molecule, such as a plasmid, which complements the deficiency serves as a selection marker whereby the growth conditions for selection may comprise a conventional complex medium.

In was found in accordance with the present invention that permanent selection using cell-viable proteins as a selectable marker can be used to over-express a protein-of-interest in mammalian cells in a cytotoxic-free medium and that proteins produced using such a method are highly suitable for pharmaceutical applications.

### SUMMARY OF THE INVENTION

According to one aspect of the present invention there is provided a method of selecting for positively-transformed cells comprising: (a) providing eukaryotic cells lacking activity of an endogenous cell viability protein; (b) transforming the eukaryotic cells with a nucleic acid construct encoding the cell viability protein or at least a functional portion thereof as well as further encoding a protein-of-interest; and (c) culturing the eukaryotic cells transformed with the nucleic acid construct under conditions such that cell viability is dependent upon normal activity of the cell viability protein thereby selecting for positively-transformed cells, wherein the cell viability protein is galactokinase 1, wherein the eukaryotic cell is a mammalian cell, wherein a nucleic acid coding for the said cell viability protein and a nucleic acid coding for said protein-of-interest are in bi-cistronic configuration with the nucleic acid sequence encoding the protein-of-interest being downstream the nucleic acid sequence encoding the cell viability protein, and wherein said culturing is effected in the presence of D-Galactose in a concentration of 0.25 mM to 0.5 mM.

According to another aspect of the present invention there is provided a cytotoxic-free eukaryotic cell culture comprising cells genetically modified to express a cell viability protein and a protein-of-interest under culturing conditions such that cell viability is dependent upon normal activity of the cell viability protein, wherein the cell viability protein is galactokinase 1, wherein the eukaryotic cell is a mammalian cell and wherein a nucleic acid coding for the said cell viability protein and a nucleic acid coding for said protein-of-interest are in bi-cistronic configuration with the nucleic acid sequence encoding the protein-of-interest being downstream the nucleic acid sequence encoding the cell viability protein, and wherein said culturing is effected in the presence of D-Galactose in a concentration of 0.25 mM to 0.5 mM.

According to yet another aspect of the present invention there is provided a method of producing a protein-of-interest, comprising: (a) transforming eukaryotic cells lacking activity of an endogenous cell viability protein with a nucleic acid construct encoding the protein-of-interest and the cell viability protein or at least a functional portion thereof; (b) culturing the eukaryotic cells transformed with the nucleic acid construct under conditions such that cell viability is dependent upon normal activity of the cell viability protein thereby selecting for positively-transformed eukaryotic cells; and (c) purifying the protein-of-interest from the positively-transformed eukaryotic cells or a culture medium thereof, wherein the cell viability protein is galactokinase 1, wherein the eukaryotic cell is a mammalian cell and wherein a nucleic acid coding for the said cell viability protein and a nucleic acid coding for said protein-of-interest are in bi-cistronic configuration with the nucleic acid sequence encoding the protein-of-interest being downstream the nucleic acid sequence encoding the cell viability protein, and wherein said culturing is effected in the presence of D-Galactose in a concentration of 0.25 mM to 0.5 mM.

According to further features in the described preferred embodiments the protein-of-interest is selected from the group consisting of a growth factor, a hormone, a cytokine, an extracellular protein, a cell adhesion protein, and a cell signaling protein.

According to still further features in the described preferred embodiments the growth factor is selected from the group consisting of Epidermal Growth Factor, transforming growth factor-beta, fibroblast growth factor-acidic, fibroblast growth factor-basic, erythropoietin, thrombopoietin, hepatocyte growth factor, insulin-like growth factor-I, insulin-like growth factor-II, Interferon-gamma, and platelet-derived growth factor.

According to still further features in the described preferred embodiments the hormone is selected from the group consisting of prolactin, parathyroid hormone, Gastrin, leptin, growth hormone, insulin and CG-β.

According to still further features in the described preferred embodiments the cytokine is selected from the group consisting of SDF-1α, IL-7, IL-10, IL-20 and IL-19.

According to still further features in the described preferred embodiments the extracellular protein is selected from the group consisting of fibrinogen, Collagen, fibronectin, vimentin, microtubule-associated protein 1b, Neurite outgrowth factor (NOF), bacterial cellulose (BC), and laminin.

According to still further features in the described preferred embodiments the cell adhesion protein is selected from the group consisting of cell adhesion proteins include integrin, intercellular adhesion molecule (ICAM) 1, N-CAM, cadherin, tenascin, gicerin, and nerve injury induced protein 2 (ninjurin2).

According to still further features in the described preferred embodiments the cell signaling protein is selected from the group consisting of p38 mitogen-activated protein kinase, nuclear factor kappaB, Raf kinase inhibitor protein (RKIP), Raf-1, MEK, Protein kinase C (PKC), phosphoinositide-3-kinase gamma, receptor tyrosine kinases, heterotrimeric G-proteins, Caveolin-3, and 14-3-3 proteins.

According to still further features in the described preferred embodiments the nucleic acid construct includes a nucleic acid sequence as set forth in SEQ ID NO:3.

According to still further features in the described preferred embodiments the concentration of the D-Galactose is 0.25 mM.

The present invention successfully addresses the shortcomings of the presently known configurations by providing a method of producing a protein-of-interest using a permanent selection in a cytotoxic-free culturing conditions.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

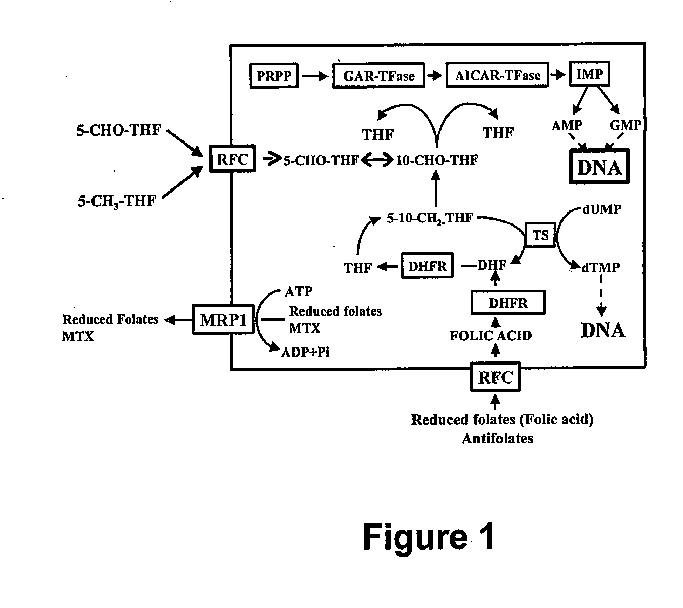

FIG. 1 is a schematic illustration depicting the metabolism of reduced folates. PRPP - 5-phosphoribosyl-1-pyrophosphate; GAR-TFase - Glycineamide ribonucleotide transformylase; AICAR-TFase - 5-Aminoimidazole-4-carboxamide ribonucleotide transformylase; MRP-1 - Multidrug resistance protein 1; TS - Thymidylate synthase; IMP - Inosine monophosphate; TS - thymidylate synthase; DHFR - dihydrofolate reductase; RFC - reduced folate carrier;

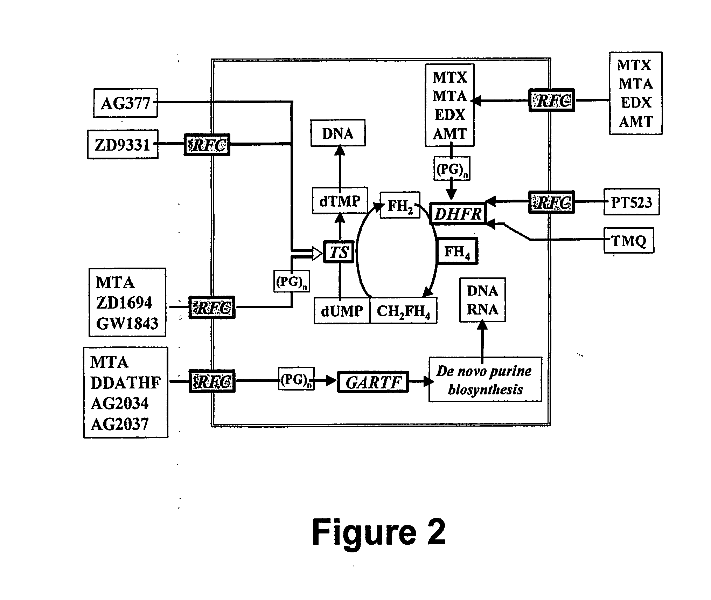

FIG. 2 is a schematic illustration depicting antifolate inhibition of folate metabolism. Folate metabolism inhibitors that enter the cell via RFC are marked in green; folate metabolism inhibitors that enter the cell by diffusion via the cell membrane are marked in brown; inhibited enzymes are marked in red. MTX-methotrexate; MTA - multitargeted antifolate; EDX - edatrexate; AMT - Aminopetrin; TMQ - Trimetrexate;; DDATHF - 5,10-Dideaza-5,6,7,8-tetrahydrofolic acid.

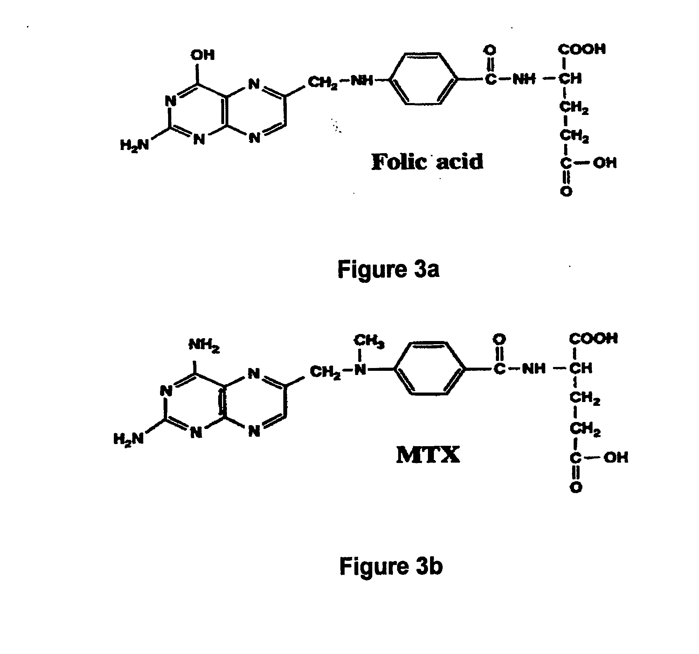

FIGs. 3a-b are schematic illustrations depicting the chemical formulas of folic acid (Figure 3a) and MTX (Figure 3b).

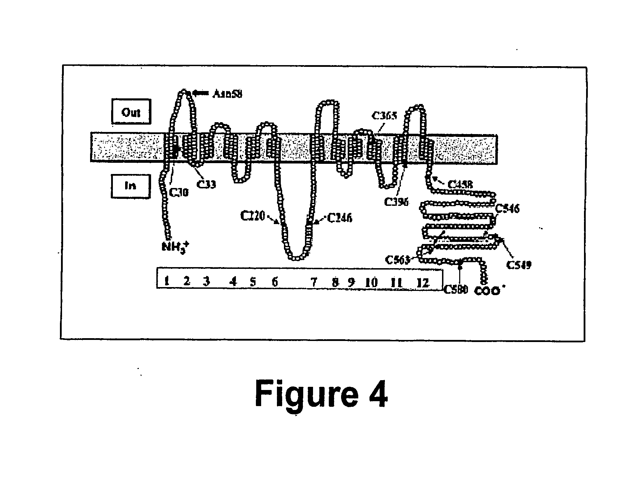

FIG. 4 is schematic illustration depicting a topology model for hRFC. The topology model for hRFC depicts 12 transmembrane domains (TMDs), internally orientated N- and C-termini and an externally orientated N-glycosylation site at Asn58. Also shown are the positions of the 11 cysteine residues in the hRFC primary sequence. The C-terminal 56 amino acids, include four cysteine residues (adopted from Cao and Matherly, 2003).

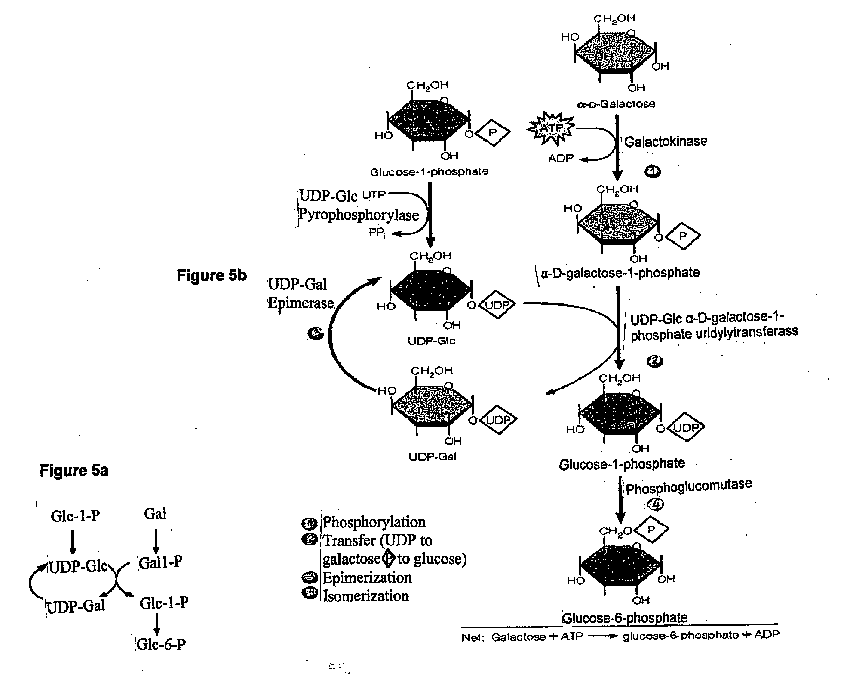

FIG. 5 is a schematic illustration depicting the metabolic conversion of galactose to glucose-6-phosphate.

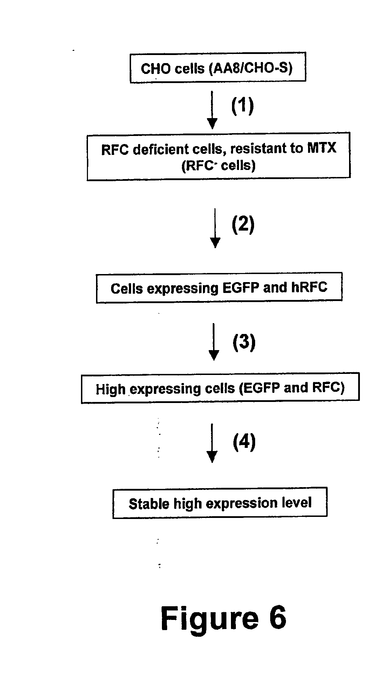

FIG. 6 is a schematic illustration depicting the permanent selection strategy with the RFC Gene. (1) Treatment with MTX for isolation of RFC- cells; (2) Transfection with an expression vector carrying EGFP (reporter gene) linked to the hRFC gene in a bi-cistronic configuration; (3) Growth in selective concentrations of a reduced folate (leucovorin) and G418; (4) Maintenance of high expression level in selective concentrations of leucovorin in the absence of G418 - Non Toxic Permanent Selection; GFP was used as the gene of interest in this study;

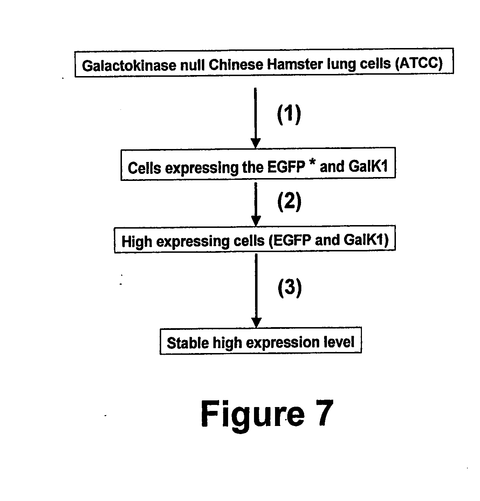

FIG. 7 is a schematic illustration depicting the permanent selection strategy with the GalK1 Gene. (1) Transfection with expression vector carrying the EGFG (reporter gene) linked to the human GalK1 gene in a bi-cistronic configuration; (2) Growth in the absence of glucose and in selective galactose concentrations and G418; (3) Maintenance of high expression level in selective galactose concentrations in the absence of G418; Non toxic permamnent selection; GFP was used as the gene of interest in this study.

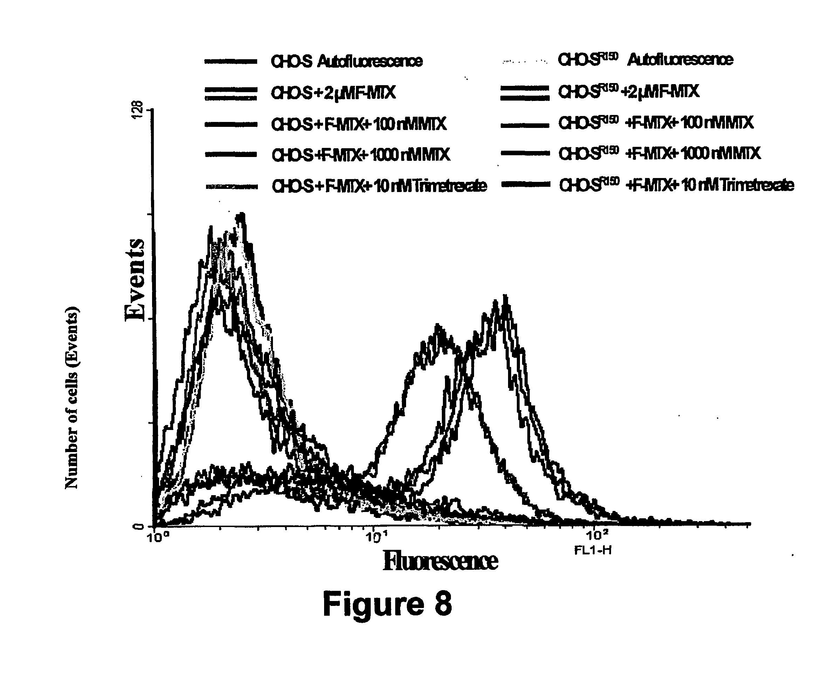

FIG. 8 is a FACS analysis depicting antifolate displacement of F-MTX fluorescence in RFC loss-of-function CHO-S cells. CHO-S and RFC deficient CHO-S cells (CHO-SR0.15 established by exposure to 150 nM MTX), were stained with F-MTX (a fluorescent MTX analogue) following which the cells were incubated with various concentrations of MTX or TMQ (Trimetrexate, a lipid- soluble antifolate that is RFC-independent for its entry). Displacement of F-MTX indicates functionally active RFC. Retention of F-MTX fluorescence after competition with 100 nM MTX concentrations in CHO-SR0.15 suggests an MTX transport defect, *i.e.* loss of RFC function.

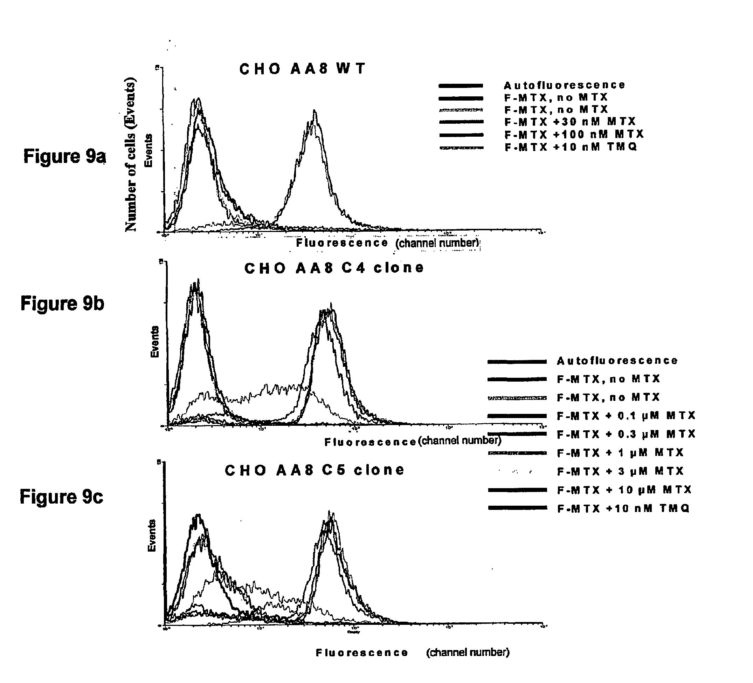

FIGs. 9a-c are FACS analyses depicting antifolate displacement of F-MTX fluorescence in RFC loss-of-function CHO AA8 Cells. Wild type CHO AA8 cells and RFC deficient clones C4 and C5 were stained with F-MTX (fluorescent MTX analogue) following which they were subjected to various concentrations of MTX or TMQ (a lipid- soluble antifolate that is RFC-independent for its entry). Displacement of F-MTX indicates functionally active RFC. Retention of F-MTX fluorescence after competition with high MTX concentrations is indicative of MTX transport defect, i.e. loss of RFC function.

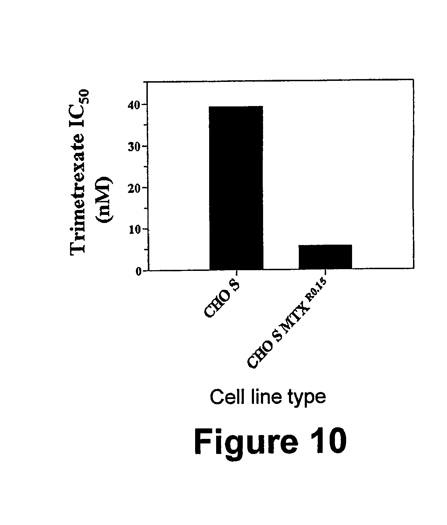

FIG. 10 is a bar graph illustrating TMQ hypersensitivity of parental or CHO-SR0.15 cells. Cells were incubated for 3 days in the presence of growth medium and various concentrations of TMQ, and the survival of cells and the 50 % inhibitory concentrations of TMQ were determined. Note the 8-fold hypersensitivity of CHO-SR0.15 cells as compared with parental CHO cells.

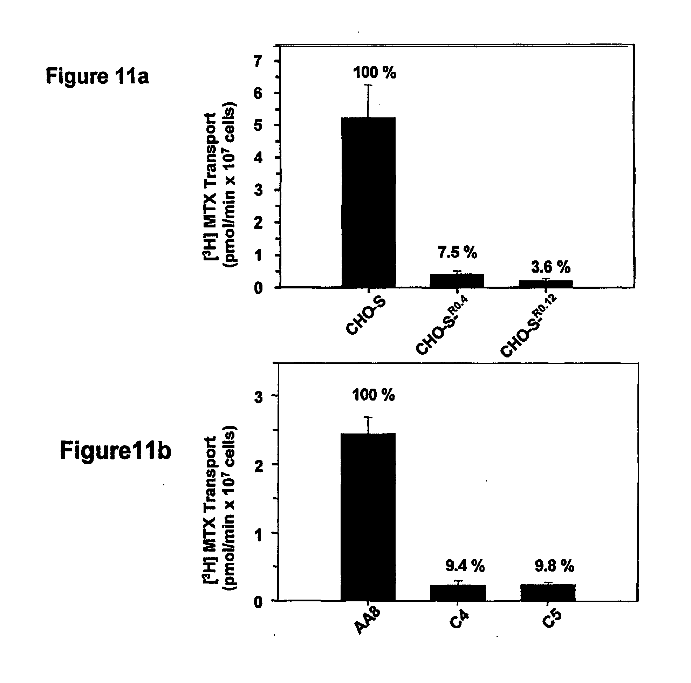

FIGs. 11a-b are bar graphs depicting loss of MTX transport in antifolate-resistant sublines. [³H]MTX transport rates were determined in parental and antifolate-resistant cells. Cells were incubated with 2 µM MTX for 3 minutes, following which the level of radiolabeled MTX uptake was determined by scintillation counting. CHO-S-R0.04 and CHO-S-R0.12are CHO-S RFC deficient cells obtained by stepwise exposure of cells up to 40 nM and 120 nM MTX respectively. C4 and C5 are CHO AA8 clones obtained by treatment with 150 nM MTX. Note the poor transport of [³H]MTX in CHO S MTXR cells as well as in CHO C4 and C5 cells, which is indicative of the loss of RFC function.

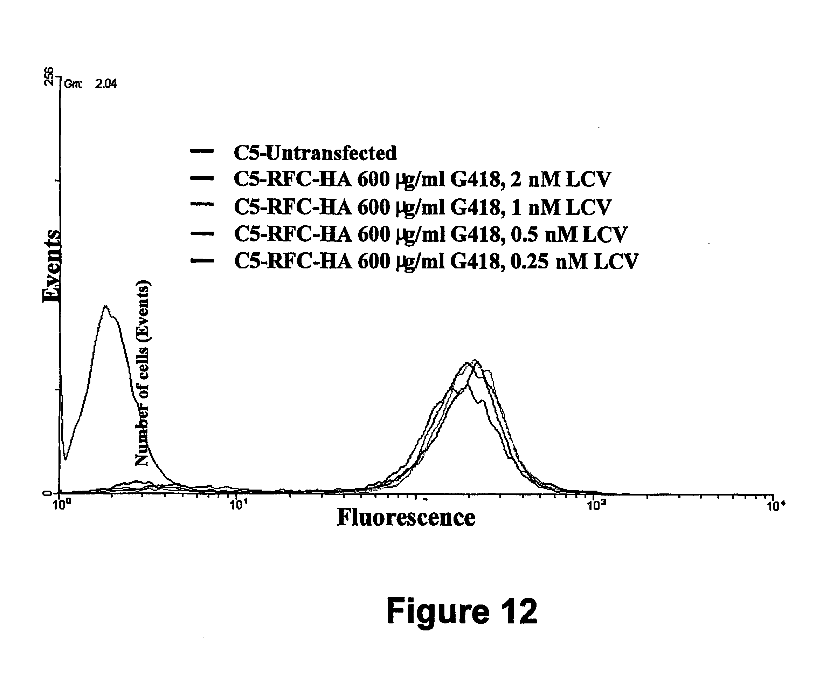

FIG. 12 is FACS analysis depicting EGFP expression in CHO C5 cells transfected with RFC-HA. Flow cytometric analysis of non-transfected CHO C5 cells and their RFC-HA transfectants doubly selected for six weeks in medium containing 600 µg/ml G418 and 0.25-2 nM leucovorin (LCV). The transfected cells appear to homogeneously express EGFP at all leucovorin concentrations.

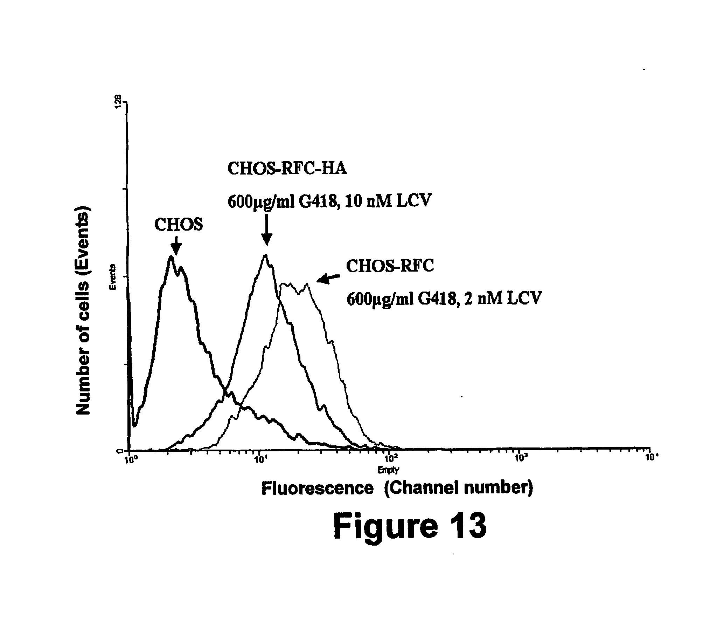

FIG. 13 is FACS analysis depicting EGFP expression in CHO-S cells transfected with RFC and RFC-HA. Representative flow cytometric analysis of EGFP fluorescence of non-transfected CHO-SR0.15 cells as well as their RFC or RFC-HA transfectants, doubly selected for two weeks in a medium containing 600 µg/ml G418 and 2-10 nM leucovorin. The CHO-SR0.15 transfectant population was homogenous and displayed a 5-10-fold increase in EGFP fluorescence relative to non-transfected cells.

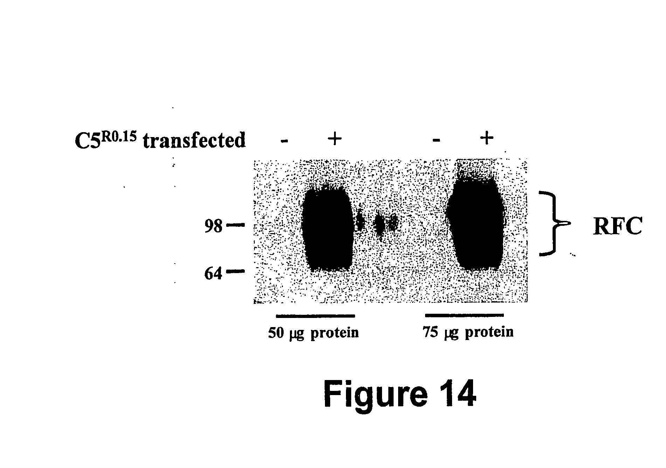

FIG. 14 is Western Blot analysis depicting the expression of RFC-HA in transfected CHO C5 Cells. Triton X-100-soluble proteins were first extracted from CHO C5 (-) cells and their RFC-HA transfectants (+) grown for four weeks in growth medium containing 600 µg/ml G-418 and 2 nM leucovorin. Proteins were then separated by SDS polyacrylamide gel electrophoresis, transferred to a nylon membrane and reacted with a monoclonal antibody to the HA tag. The membranes were developed using a standard enhanced chemiluminescence (ECL) procedure. Note the specific expression of the HA tag in RFC-HA transfected cells.

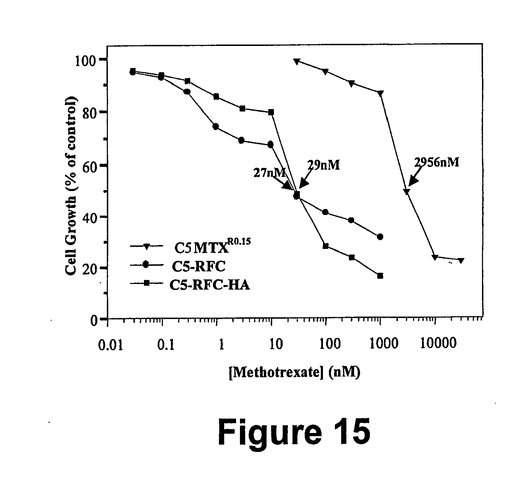

FIG. 15 is a graph depicting MTX growth inhibition in RFC transfected cells. Shown is the cytotoxicity of MTX to C5R0.15 cells (RFC deficient) and their RFC-HA transfectants. Cells were incubated for 3 days in various concentrations of MTX, following which cell survival was measured using trypan blue exclusion and the 50 % inhibitory concentrations were determined (indicated by the arrows). Note the 100-fold increase in MTX sensitivity in both the RFC and RFC-HA transfectants.

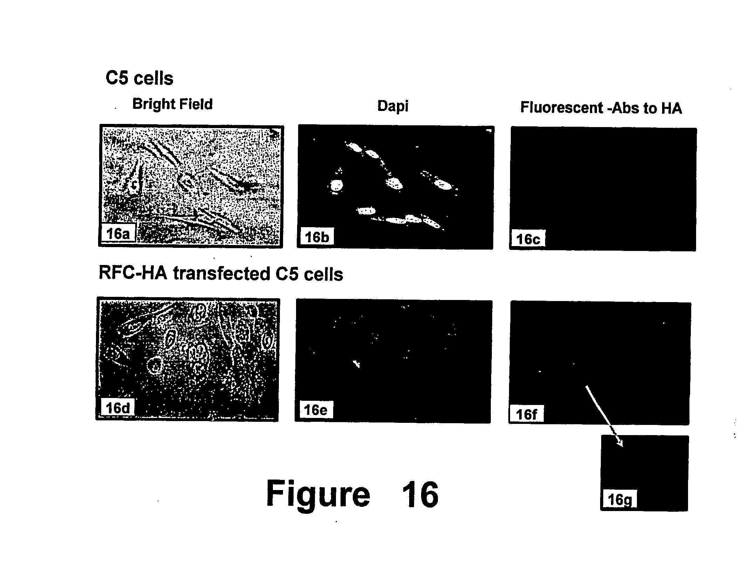

FIGs. 16a-g are fluorescence images depicting immunofluorescence staining of RFC. C5 parental cells or C5 RFC-HA transfected cells were cultured in the presence of 1 nM leucovorin and 600 and mg/ml G418 were subjected to immunofluorescence staining. Cells were fixed with 4 % formaldehyde, treated with lysis buffer and stained with MAbs to the hemagglutinin (HA) tag followed by secondary fluorescein-conjugated antibodies. Dapi nucleic acid staining shows cell nuclei. Figures 16a-c - untransfected C5 cells (RFC-, negative control); Figures 16d-g - RFC-HA C5 cells. Note the green fluorescence staining observed following HA immunostaining in the RFC-HA C5 transfected cells (Figures 16f and g) as compared with untransfected C5 cells (Figure 16c). Figure 16g depicts a magnification of a stained cell of Figure 16f.

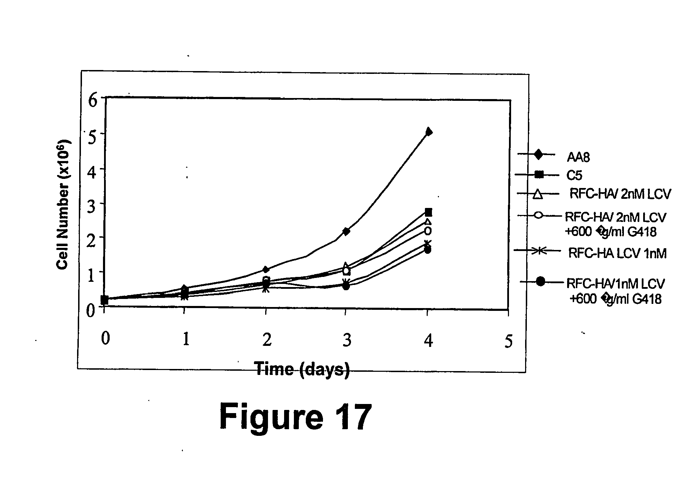

FIG. 17 is a graph depicting the growth of hRFC transfected cells under selective growth conditions. 2x105 cells were seeded in 30 mm petri dishes (5 ml medium/dish). Cell concentrations were determined daily for a total of 4 days, without medium refreshment. hRFC-HA transfectants were grown in selective medium containing dialyzed FBS, 1 or 2 nM leucovorin (LCV) in the presence of 0.6 mg/ml G418 or after the G418 was removed for three weeks. Parental RFC- AA8 and C5 cells, which could not grow in the selective medium, were grown in non-selective medium (RPMI) containing 2.3 µM folic acid and supplemented with 10 % FBS. Doubling time of the hRFC-HA transfectants was ~28 hours.

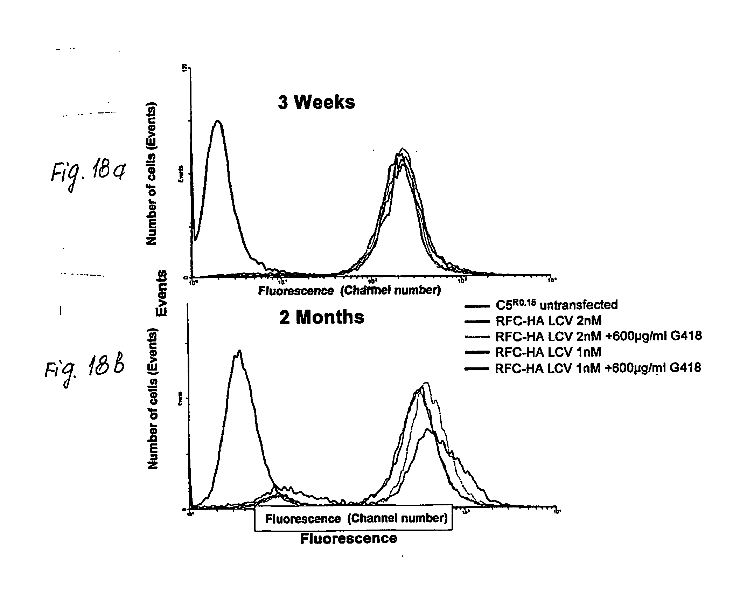

FIGs. 18a-b are FACS analyses depicting the stability of EGFP expression under permanent selection conditions with RFC. Shown is a flow cytometric analysis of enhanced green fluorescence protein (EGFP) expression in hRFC-HA transfected C5 cells. hRFC-HA transfectants were grown in selective medium containing dialyzed FBS, 1 or 2 nM leucovorin (LCV) in the presence of 600 µg/ml G-418, or after G-418 removal. Parental RFC- C5 cells (negative control), which could not grow in the selective medium, were grown in non-selective medium containing 2.3 µM folic acid and supplemented with 10 % FBS.

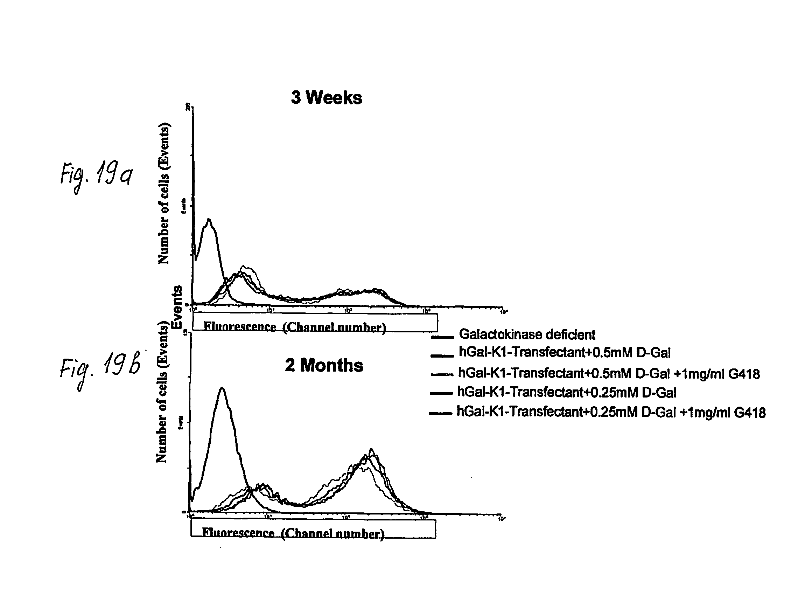

FIGs. 19a-b are FACS analyses depicting stability of EGFP expression under permanent selection conditions with GalK1. Shown is a flow cytometric analysis of EGFP expression in Galactokinase deficient Chinese hamster cells transfected with the hGal-K1 - EGFP bicistronic vector, growing under selection at sub-millimolar galactose concentrations in the absence or presence of G-418 for three weeks (Figure 19a) or two months (Figure 19b). Galactokinase deficient cells were grown in non-selective medium.

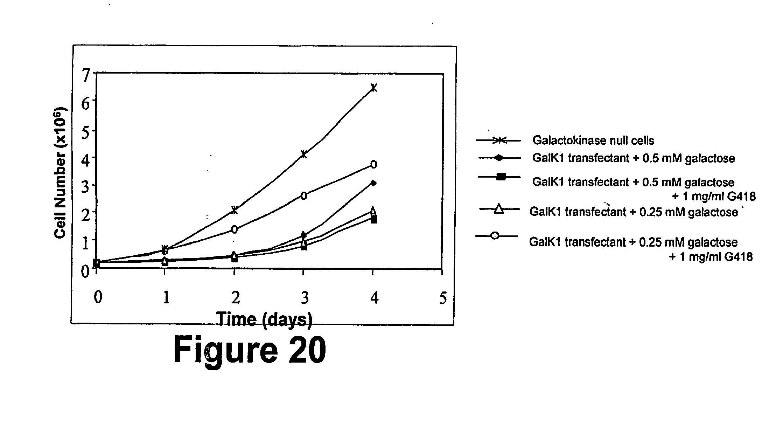

FIG. 20 is a graph depicting the growth of GalK1 transfected cells under selective growth conditions. 2x105 cells were seeded in 30 mm petri dishes (5 ml medium/dish). Cell concentrations were determined daily for a total of 4 days without medium replacement. GalK1 transfectants were grown in selective medium (RPMI-1640 supplemented with 10 % dialyzed FBS, 0.25-0.5 mM D-galactose) in the presence of 1 mg/ml G418 or after G418 was removed for three weeks. Parental Galactokinase deficient Chinese hamster cells were grown in non-selective medium (RPMI-1640 containing 5 mM D-glucose and supplemented with 10 % fetal calf serum). Doubling time of the GalK1 transfectants in the selective medium was ~28 hours.

### DESCRIPTION OF THE PREFERRED EMBODIMENTS

The present invention is of a method of producing proteins in mammalian cells using a permanent selection in the absence of cytotoxic drugs. Specifically, the present invention can be used to produce large quantities of highly pure human proteins which are suitable for pharmaceutical applications.

The principles and operation of the method of producing proteins according to the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Human growth factors and hormones are widely used in the treatment of genetic or infectious diseases. However, the purification of human proteins from human body sources such as blood is complicated, labor-intense and expensive. In addition, proteins purified from human or animal cells may be contaminated with various pathogens such as the HIV or Hepatitis viruses and are therefore not suitable for use in pharmaceutical applicaitons.

To overcome such limitations, recombinant expression systems have been developed. Expression of recombinant human proteins from mammalian expression system enables correct protein folding and appropriate post-translational modifications. However, expression of human proteins using common mammalian expression systems often results in low protein yields as well as in traces of cytotoxic drugs [e.g., methotrexate (MTX)] which are used to positively select for the transfected cells.

It was found that permanent selection using a cell-viable protein as a selectable marker can be used to over-express a protein-of-interest in mammalian cells in the absence of cytotoxic drugs and that proteins produced using such a method are highly suitable for pharmaceutical applications.

As is shown in Examples 1-3 of the Examples section which follows, co-transfection of reduced folate carrier (RFC) - deficient cells with a bi-cistronic expression vector including the RFC gene and a reporter gene (e.g., EGFP) followed by a gradual decrease in the concentrations of leucovorin (a reduced folate) resulted in over-expression of the RFC gene and a significant expression of the reporter gene in a medium free of any cytotoxic drugs. Moreover, as is shown in Examples 1, 2 and 4 of the Examples section which follows, co-transfection of GalK 1 - deficient cells with a bi-cistronic expression vector including the GalK1 gene and a reporter gene (e.g., EGFP) followed by a selection in a growth medium containing 5 mM D-Galactose as the sole hexose source resulted in over-expression of the GalK1 and EGFP proteins in the absence of any cytotoxic drugs.

Thus, provided herewith is a method of selecting for positively-transformed cells.

As used herein the phrase "positively-transformed cells" refers to cells being positive for a genetic transformation, i.e., cells in which an exogenous DNA molecule (*i.e.,* a polynucleotide) is either integrated into a genome of the cell or present in the cell nucleus as an extrachromosomal replication unit. Methods of transforming cells are known in the arts and are further described hereinbelow.

The method is effected by (i) providing eukaryotic cells lacking activity of an endogenous cell viability protein; (ii) transforming the eukaryotic cells with a nucleic acid construct encoding the cell viability protein or at least a functional portion thereof; and (iii) culturing the eukaryotic cells transformed with the nucleic acid construct under conditions such that cell viability is dependent upon normal activity of the cell viability protein thereby selecting for positively-transformed cells.

As used herein the phrase "cell viability protein" refers to any protein which is essential for cell growth, i.e., in the absence of which the cell is unable to grow. Cell growth is dependent upon the assimilation, uptake, incorporation and biosynthesis of nutrients such as amino acids, nucleic acids, fatty acids, carbohydrates, vitamins and the like, which are essential for biosynthesis of cell components (e.g., protein, DNA, RNA, carbohydrates and fat) and/or cell compartments (e.g., membranes, organelles). Thus, a cell viability protein is any protein which is essential for cell growth as described hereinabove. Preferably, the cell viability protein is the reduced folate carrier protein (RFC), the Galactokinase 1 protein (GalK1), the thymidine kinase protein, the adenosine kinase protein, tryptophan synthase, histidinol dehydrogenase and glutamine synthetase.

The phrase "functional portion" as used herein refers to part of the cell viability protein (*i.e.*, a polypeptide) which exhibits functional properties of the protein such as binding to a substrate. Preferably, the functional portion of the reduced folate carrier is a polypeptide sequence including amino acids 23-434 (region of folate carrier) as set forth in SEQ ID NO:1 (GenBank Accession No. AAB35058), more preferably, the functional portion of the reduced folate carrier of the present invention is a polypeptide sequence including amino acids 1-591 as set forth in SEQ ID NO:1. According to preferred embodiments of the present invention, the functional portion of the Galactokinase 1 protein of the present invention is a polypeptide sequence including amino acids 1-392 as set forth in SEQ ID NO:2 (GenBank Accession No. NP_000145).

Eukaryotic cells can be any cells having a highly developed and complex nucleus which is surrounded by a nuclear envelope consisting of two membranes. Eukaryotic cells can be yeast cells, insect cells, plant cells and mammalian cells. The eukaryotic cells of the present invention are mammalian cells such as Chinese hamster cells (CHO cells), Chinese Hamster lung cells, Baby Hamster Kidney cells, COS-1 cells, NIH/3T3 cells, Caco-2 cells, 293T cells, HeLa cells, CCRF-CEM, L1210.

According to the method of the present invention the eukaryotic cells of the present invention lack the cell viability protein of the present invention. Examples for such cells include CHO AA8 cells lacking the RFC transporter activity (Assaraf and Schimke, 1987), Chinese Hamster lung cells deficient in the galactokinase (GalK) enzyme (ATCC; CRL-1657), thymidine kinase deficient mouse cells [Kaufman ER and Davidson RL, 1975, Somatic Cell Genet. 1(2): 153-63], adenosine kinase deficient Baby Hamster Kidney cells [Mittal RA, et al., 2000; Biofactors. 11(4): 247-56], Tryptophan synthase-deficient and Histidinol dehydrogenase-deficient CHO cells (Hartman SC and Mulligan RC, 1988, Proc. Natl. Acad. Sci. USA 85: 8047-8051).

Methods of obtaining cells deficient in a cell viability protein are known in the art. For example, cells lacking the RFC transport activity can be obtained by subjecting cells to high concentrations of a folate antagonist [e.g., methotrexate (MTX)] and isolating surviving cells lacking the RFC transport activity, essentially as described in Example 2 of the Examples section which follows. Briefly, CHO AA8 cells (5 x 10⁵ /10 cm Petri dish, in the presence of 20 ml medium) are grown as a monolayer in αMEM growth medium containing 2.3 µM folic acid supplemented with 10 % fetal bovine serum (GIBCO), 2 mM glutamine and antibiotics. For MTX selection, cells are exposed to 75 nM and 150 nM MTX (Sigma) which corresponds to -5- and 10-fold, respectively, of the 50 % lethal dose (LD₅₀). Alternatively, a stepwise selection can be carried out by gradually increasing MTX concentrations from 10 nM, through 40 nM, 120 nM and 150 nM in the medium over a period of ~ five months.

Cells lacking the Galactokinase 1 protein can be obtained by exposing cells to the toxic galactose analog, 2-deoxy-D-galactose (2-DOG), essentially as described in Zaret KS and Stevens KA, Mol Cell Biol. 1990;10(9): 4582-9.

The cells of the present invention (which lack the activity of a cell viability protein) are transformed with a nucleic acid construct encoding the cell viability protein of the present invention (e.g., reduced folate carrier, Galactokinase 1, thymidine kinase, and/or adenosine kinase).

Reduced folate carrier has been cloned from human (GenBank Accession No. AAB35058), mouse (GenBank Accession No. AAC53287) and rat (GenBank Accession No. AAC61788); Galactokinase 1 has been cloned from human (GenBank Accession No. NP_000145), mouse (GenBank Accession No. NP_058601) and rat (GenBank Accession No. XP_213528); thymidine kinase has been cloned from human (GenBank Accession No. AAN73847), mouse (GenBank Accession No. AAD35091) and various bacteria and viruses; adenosine kinase has been cloned from human (GenBank Accession No. AAP35434), mouse (GenBank Accession No. AAH09659), rat (GenBank Accession No. AAH81712) and many other organisms. Thus, coding sequences information for reduced folate carrier, Galactokinase 1, thymidine kinase and/or adenosine kinase are available from several databases including the GenBank database available through http://www4.ncbi.nlm.nih.gov/.

To express an exogenous cell viability protein (e.g., reduced folate carrier, Galactokinase 1) in mammalian cells, a polynucleotide sequence encoding the cell viability protein [e.g., GenBank Accession No. S78996 (SEQ ID NO:4) for human reduced folate carrier; GenBank Accession No. NM_000154 (SEQ ID NO:3) for human Galactokinase 1] is preferably ligated into a nucleic acid construct suitable for mammalian cell expression. Such a nucleic acid construct includes a promoter sequence for directing transcription of the polynucleotide sequence in the cell in a constitutive or inducible manner.

It will be appreciated that the nucleic acid construct of the present invention can also utilize a nucleic acid sequence encoding a cell viability protein homologue which exhibits the desired activity (e.g., transport of Galactokinase activity). Such nucleic acid sequences can be, for example, at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95 %, at least 96 %, at least 97 %, at least 98 %, at least 99 % or 100 % identical to SEQ ID NO:3, as determined using the BestFit software of the Wisconsin sequence analysis package, utilizing the Smith and Waterman algorithm, where gap weight equals 50, length weight equals 3, average match equals 10 and average mismatch equals -9.

The nucleic acid construct of the present invention further includes a nucleic acid sequence encoding a protein-of-interest.

As used herein the phrase "protein-of-interest" refers to any polypeptide which anyone skilled in the art would produce in the eukaryotic cells of the present invention. Preferably, the protein-of-interest of the present invention can be used for pharmaceutical and/or basic science purposes. According to preferred embodiments of the present invention the protein-of-interest of the present invention can be a growth factor, a cytokine and/or a hormone which is used for the treatment of various disorders, as well as an extracellular matrix protein, a cell adhesion protein and/or a cell signaling protein which may be used for tissue regeneration applications.

Non-limiting examples of growth factors include Epidermal Growth Factor (GenBank Accession No. NP_001954), transforming growth factor-beta (GenBank Accession No. NP_000651), fibroblast growth factor-acidic (GenBank Accession No. NP_000791), fibroblast growth factor-basic (GenBank Accession No. NP_001997), erythropoietin (GenBank Accession No. NP_000790), thrombopoietin (GenBank Accession No. NP_000451), hepatocyte growth factor (GenBank Accession No. NP_000592), insulin-like growth factor-I (GenBank Accession No. NP_000609), insulin-like growth factor-II (GenBank Accession No. NP_000603), Interferon-gamma (GenBank Accession No. NP_000610), and platelet-derived growth factor (GenBank Accession No. NP_079484).

Non-limiting examples of hormones include prolactin (GenBank Accession No. AAH15850), parathyroid hormone (GenBank Accession No. AAK34950), Gastrin (GenBank Accession No. AAA52520), leptin (GenBank Accession No. EAL24315), growth hormone (GenBank Accession No. AAA98618), insulin (GenBank Accession No. AAN39451) and CG-β (GenBank Accession No. P01233).

Non-limiting examples of cytokines include SDF-1α (GenBank Accession No. AAA40100), IL-7 (GenBank Accession No. NP_000871), IL-10 (GenBank Accession No. NP_000563), IL-20 (GenBank Accession No. NP_061194) and IL-19 (GenBank Accession No. NP_715639).

Non-limiting examples of extracellular protein include fibrinogen (GenBank Accession No. NP_000499), Collagen (GenBank Accession No. NP_000079), fibronectin (GenBank Accession No. NP_002017), vimentin (GenBank Accession No. NP_003371), microtubule-associated protein 1b (GenBank Accession No. NP_005900) (Theodosis DT. 2002; Front Neuroendocrinol. 23: 101-35), Neurite outgrowth factor (NOF) (GenBank Accession No. P21741) (Tsukamoto Y, et al., 2001; Histol. Histopathol. 16: 563-71), bacterial cellulose (BC) (GenBank Accession No. NP_625477), and laminin (GenBank Accession No. NP_000218).

Non-limiting examples of cell adhesion proteins include integrin (GenBank Accession No. NP_002202) (Stefanidakis M, et al., 2003; J Biol Chem. 278: 34674-84), intercellular adhesion molecule (ICAM) 1 (GenBank Accession No. NP_000192) (van de Stolpe A and van der Saag PT. 1996; J. Mol. Med. 74: 13-33), N-CAM GenBank Accession No. NP_000606), cadherin (GenBank Accession No. NP_004351), tenascin (GenBank Accession No. NP_061978) (Joshi P, et al., 1993; J. Cell Sci. 106: 389-400), gicerin (GenBank Accession No. NP_006491), and nerve injury induced protein 2 (ninjurin2) (GenBank Accession No. NP_067606) (Araki T and Milbrandt J. 2000; J. Neurosci. 20: 187-95).

Non-limiting examples of cell signaling proteins include p38 mitogen-activated protein kinase (GenBank Accession No. NP_002736), nuclear factor kappaB (GenBank Accession No. NP_003989), Raf kinase inhibitor protein (RKIP) (GenBank Accession No. XP_497846), Raf-1 (GenBank Accession No. NP_002871), MEK (GenBank Accession No. NP_002746), Protein kinase C (PKC) (GenBank Accession No. NP_002728), phosphoinositide-3-kinase gamma (GenBank Accession No. NP_002640), receptor tyrosine kinases [e.g., insulin receptor (GenBank Accession No. NP_000199)], heterotrimeric G-proteins [e.g., Galpha(i) (GenBank Accession No. NP_002060), Galpha(s) NP_000507 and Galpha(q) (GenBank Accession No. NP_002063)], Caveolin-3 (GenBank Accession No. NP_001225), and 14-3-3 proteins (GenBank Accession No. NP_003397).

Constitutive promoters suitable for use with the present invention are promoter sequences which are active under most environmental conditions and most types of cells such as the cytomegalovirus (CMV) and Rous sarcoma virus (RSV). Inducible promoters suitable for use with the present invention include for example the inducible promoters of the metalothionein genes (MT I and MTII) (Majumdar S et al., (2003) J. Biol. Chem. 278: 26216-26226) and the tetracycline-inducible promoter [Zabala M, et al., Cancer Res. 2004, 64(8): 2799-804].

The nucleic acid construct (also referred to herein as an "expression vector") of the present invention includes additional sequences which render this vector suitable for replication and integration in prokaryotes, eukaryotes, or preferably both (e.g., shuttle vectors). In addition, a typical cloning vector may also contain a transcription and translation initiation sequence, transcription and translation terminator and a polyadenylation signal.

Eukaryotic promoters typically contain two types of recognition sequences, the TATA box and upstream promoter elements. The TATA box, located 25-30 base pairs upstream of the transcription initiation site, is thought to be involved in directing RNA polymerase to begin RNA synthesis. The other upstream promoter elements determine the rate at which transcription is initiated.

Enhancer elements can stimulate transcription up to 1,000 fold from linked homologous or heterologous promoters. Enhancers are active when placed downstream or upstream from the transcription initiation site. Many enhancer elements derived from viruses have a broad host range and are active in a variety of tissues. For example, the SV40 early gene enhancer is suitable for many cell types. Other enhancer/promoter combinations that are suitable for the present invention include those derived from polyoma virus, human or murine cytomegalovirus (CMV), the long term repeat from various retroviruses such as murine leukemia virus, murine or Rous sarcoma virus and HIV. See, Enhancers and Eukaryotic Expression, Cold Spring Harbor Press, Cold Spring Harbor, N.Y. 1983.

In the construction of the expression vector, the promoter is preferably positioned approximately the same distance from the heterologous transcription start site as it is from the transcription start site in its natural setting. As is known in the art, however, some variation in this distance can be accommodated without loss of promoter function.

Polyadenylation sequences can also be added to the expression vector in order to increase the efficiency of translation of the cell viability protein and/or protein-of-interest. Two distinct sequence elements are required for accurate and efficient polyadenylation: GU or U rich sequences located downstream from the polyadenylation site and a highly conserved sequence of six nucleotides, AAUAAA, located 11-30 nucleotides upstream. Termination and polyadenylation signals that are suitable for the present invention include those derived from SV40.

In addition to the elements already described, the expression vector of the present invention may typically contain other specialized elements intended to increase the level of expression of cloned nucleic acids or to facilitate the identification of cells that carry the recombinant DNA. For example, a number of animal viruses contain DNA sequences that promote the extra chromosomal replication of the viral genome in permissive cell types. Plasmids bearing these viral replicons are replicated episomally as long as the appropriate factors are provided by genes either carried on the plasmid or with the genome of the host cell.

The vector may or may not include a eukaryotic replicon. If a eukaryotic replicon is present, then the vector is amplifiable in eukaryotic cells using the appropriate selectable marker. If the vector does not comprise a eukaryotic replicon, no episomal amplification is possible. Instead, the recombinant DNA integrates into the genome of the engineered cell, where the promoter directs expression of the desired nucleic acid.

The expression vector of the present invention can further include additional polynucleotide sequences that allow, for example, the translation of several proteins from a single mRNA such as an internal ribosome entry site (IRES) and sequences for genomic integration of the promoter-chimeric polypeptide.

According to the present invention, in the construction of the nucleic acid construct of the present invention, the nucleic acid sequence encoding the protein-of-interest is ligated downstream of the nucleic acid sequence encoding the cell viability protein. Preferably, the nucleic acid sequence encoding the protein-of interest is ligated downstream of the cell viability protein using e.g., an IRES-containing expression vector, such that expression of the protein-of-interest is under the same promoter regulating the expression of the cell viability protein.

For example, as is shown in Example 2 of the Examples section which follows, the hRFC cDNA (*i.e.,* the cell viability protein) was cloned upstream of the IRES element in the pIRES-2-EGFP vector (Clontech), *i.e.,* upstream of the nucleic acid sequence encoding the protein-of-interest (EGFP).

Examples for mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

Expression vectors containing regulatory elements from eukaryotic viruses such as retroviruses can be also used. SV40 vectors include pSVT7 and pMT2. Vectors derived from bovine papilloma virus include pBV-1MTHA, and vectors derived from Epstein Bar virus include pHEBO, and p2O5. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

As described above, viruses are very specialized infectious agents that have evolved, in many cases, to elude host defense mechanisms. Typically, viruses infect and propagate in specific cell types. The targeting specificity of viral vectors utilizes its natural specificity to specifically target predetermined cell types and thereby introduce a recombinant gene into the infected cell. Thus, the type of vector used by the present invention will depend on the cell type transformed. The ability to select suitable vectors according to the cell type transformed is well within the capabilities of the ordinary skilled artisan and as such no general description of selection consideration is provided herein. For example, bone marrow cells can be targeted using the human T cell leukemia virus type I (HTLV-I) and kidney cells may be targeted using the heterologous promoter present in the baculovirus Autographa california nucleopolyhedrovirus (AcMNPV) as described in Liang CY et al., 2004 (Arch Virol. 149: 51-60).

Recombinant viral vectors are useful for *in vivo* expression of the cell viability protein and/or the protein-of-interest of the present invention since they offer advantages such as lateral infection and targeting specificity. Lateral infection is inherent in the life cycle of, for example, retrovirus and is the process by which a single infected cell produces many progeny virions that bud off and infect neighboring cells. The result is that a large area becomes rapidly infected, most of which was not initially infected by the original viral particles. This is in contrast to vertical-type of infection in which the infectious agent spreads only through daughter progeny. Viral vectors can also be produced that are unable to spread laterally. This characteristic can be useful if the desired purpose is to introduce a specified gene into only a localized number of targeted cells.

Various methods can be used to introduce the expression vector of the present invention into stem cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

Introduction of nucleic acids by viral infection offers several advantages over other methods such as lipofection and electroporation, since higher transfection efficiency can be obtained due to the infectious nature of viruses.

Culturing according to the present invention is effected under conditions such that cell viability is dependent upon normal activity of the cell viability protein. It will be appreciated that such conditions depend on the type of cell viability protein used.

For example, if the cells are those which lack the activity of a reduced folate carrier (e.g., CHO AA8 cells), then following transfection with the nucleic acid construct encoding the reduced folate carrier (e.g., SEQ ID NO:4) the cells are cultured in a culture medium containing a reduced folate [e.g., 5-formyl-tetrahydrofolate (leucovorin) or 5-methyl-tetrahydrofolate] as a sole source of folic acid.

Preferably, such culturing conditions include leucovorin at a concentration range of 0.01 - 5 nM, more preferably, at a concentration range of 0.02 - 2 nM, most preferably, at a concentration range of 0.25 - 2 nM.

As is shown in Example 2 of the Example section which follows, selection of reduced folate carrier - expressing cells was carried out in the presence of a culture medium (RPMI-1640 medium lacking folic acid) supplemented with 10 % dialyzed fetal calf serum and containing 2 nM leucovorin as the sole folate source. Following 5 weeks in culture the cells were permanently selected in the presence of 1, 0.5, or 0.25 nM leucovorin.

Alternatively, if the cells are those which lack the activity of the Galactokinase 1 protein [e.g., GalK1-deficient cells; Zaret, 1990 (Supra)], then following transfection with the nucleic acid construct encoding the Galactokinase 1 (e.g., SEQ ID NO:3) the cells are cultured in a culture medium containing D-Galactose as a sole hexose source.

Preferably, such culturing conditions include D-Galactose at a concentration range of 0.01 - 10 mM, more preferably, at a concentration range of 0.45 - 8 mM, more preferably, at a concentration range of 0.1 - 5 mM, most preferably, at a concentration range of 0.25 - 0.5 mM.

As is shown in Example 2 of the Examples section which follows, selection of D-Galactokinase - expressing cells was carried out in the presence of a culture medium (RPMI-1640 medium lacking glucose) supplemented with 10 % dialyzed fetal calf serum and containing either 5 mM or 2 mM D-Galactose as the sole hexose source. Following three days, the cells were transferred to a culture medium containing 1 or 0.5 mM D-Galactose and were cultured under such conditions for 40 days, following which they were permanently selected in the presence of 0.25 or 0.1 mM D-Galactose as the sole hexose source.

To facilitate the selection process of the positively-transformed cells of the present invention and to prevent the growth of un-transfected cells, transfection is preferably carried out in the presence of a negative selection agent such as an antibiotic drug. Thus, immediately following transfection, transfected cells which include the antibiotic resistance gene on the expression vector (e.g., the neomycin phosphotransferase gene can be cultured in the presence of relatively high concentrations of antibiotic (e.g., 300-600 µg of G418), while un-transfected cells are unable to grow. However, it will be appreciated that once the transfected cells express sufficient levels of the cell viability protein of the present invention such a negative selection agent (e.g., G418) is un-necessary and preferably removed.

As is shown in Example 2 of the Examples section which follows, G418 was used at a concentration of 300-600 µg/ml for the first few weeks of selection, following which G418 was removed and stable transformants were cultured under cytotoxic-free culturing conditions in the presence of reduced folates (e.g., leucovorin) or D-Galactose, depending on the type of cells used.

Further provided herewith is a cytotoxic-free eukaryotic cell culture.

As used herein the phrase "cytotoxic-free" refers to the state of being free of any agent which is toxic to a cell and can interfere with cell functioning. Normal functioning can be interrupted by agents which induce, for example, abnormal cell proliferation (e.g., cancerous agents), aminoglycosides and other antibiotic agents which interfere with protein translation, DNA binding agents which inhibit gene expression and the like.

The cell culture of the present invention, as defined in the claims, includes cells which are genetically modified as described hereinabove to express the cell viability protein and the protein-of-interest of the present invention under the selective culturing conditions of the present invention.

It will be appreciated that when the nucleic acid sequences encoding the cell viability protein and the protein-of-interest are placed under the same transcription regulation in the nucleic acid construct of the present invention (e.g., using a bi-cistronic vector), similar levels of expression are expected from both nucleic acid sequences in the transfected cells. Thus, cells which express high levels of the cell viability protein are likely to express similar levels of the protein-of-interest.

Indeed, as is shown in Figures 12 and 13 and Example 3 of the Examples section which follows, flow cytometry analysis revealed 100-fold or 10-fold increase in EGFP expression in cells transfected with the RFC expression vectors and cultured in the presence of 0.25-2 nM or 2-10 nM leucovorin, respectively. Thus, the method of the present invention can be used to produce high levels of a protein-of-interest under cytotoxic-free culturing conditions.

According to yet another aspect there is provided a method of producing a protein-of-interest.

The method according to this aspect is effected by transforming the eukaryotic cells of the present invention (i.e., cells lacking the activity of an endogenous cell viability protein) with a nucleic acid construct encoding the protein-of-interest and the cell viability protein or at least a functional portion thereof; culturing the cells as described hereinabove and purifying the protein-of-interest from the transformed cells.

As used herein the phrase "purifying" refers to isolating the protein-of-interest of the present invention from the cells or cell culture of the present invention. The protein-of-interest of the present invention can be isolated as a polypeptide sequence alone, as part of a protein-lipid complex (e.g., in membrane proteins), as part of a protein-nucleic acids complex (e.g., in histone proteins), with various modifications such as carbohydrates (e.g., glycoproteins) and the like. In addition, the purified protein of the present invention can be in any form, *i.e.,* solubilized, lyophilized, crystallized and the like.

Methods of purifying proteins from eukaryotic cells are known in the arts. For example, recombinant secreted proteins can be purified from the culture medium using for example, ion exchange and reverse phase chromatography as described in WO Pat. Appl. No. 86/07594 to Por-Hsiung, L. and Strickland, T; Schneider P, Methods Enzymol. 2000; 322: 325-45. Recombinant membrane bound proteins can be purified using ionic detergents such as sodium dodecyl sulfate (SDS), urea-based solutions with non-ionic detergents such as triton X-100 and CHAPS and chaotropic agents, detergents, and organic solvents (Molloy, 2000; Santoni et al., 2000; Taylor et al., 2002; Ferro et al., 2002; Ferro et al., 2000) and DNA-bound proteins can be purified as described in Pedersen LB et al., Mol Microbiol. 1996; 20(2): 295-311.

Since the cells producing the protein-of-interest of the present invention are cultured under cytotoxic-free culturing conditions (*i.e*., under the permanent selection following the removal of antibiotics) the protein produced by such cells is highly pure and free of cytotoxic contaminants. It will be appreciated that such a protein can be used in various applications including therapeutic applications in which a highly pure, cytotoxic-free protein-of-interest is administered to an individual in need thereof.

The protein-of-interest of the present invention can be administered to an individual per se, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the protein-of-interest accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

Suitable routes of administration may, for example, include oral, rectal, transmucosal, especially transnasal, intestinal or parenteral delivery, including intramuscular, subcutaneous and intramedullary injections as well as intrathecal, direct intraventricular, intravenous, inrtaperitoneal, intranasal, or intraocular injections.

Alternately, one may administer the pharmaceutical composition in a local rather than systemic manner, for example, via injection of the pharmaceutical composition directly into a tissue region of a patient.

Pharmaceutical compositions may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients (nucleic acid construct) effective to prevent, alleviate or ameliorate symptoms of a disorder (e.g., ischemia) or prolong the survival of the subject being treated.

Determination of a therapeutically effective amount is well within the capability of those skilled in the art, especially in light of the detailed disclosure provided herein.

For any preparation, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.l).

Dosage amount and interval may be adjusted individually to provide sufficient levels of the active ingredient to achieve the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation formulated in a compatible pharmaceutical carrier may also be prepared, placed in an appropriate container, and labeled for treatment of an indicated condition, as if further detailed above.

As used herein the term "about" refers to ± 10 %.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., Ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (Eds.) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., Ed. (1994); "Culture of Animal Cells - A Manual of Basic Technique" by Freshney, Wiley-Liss, N. Y. (1994), Third Edition; "Current Protocols in Immunology" Volumes I-III Coligan J. E., Ed. (1994); Stites et al. (Eds.), "Basic and Clinical Immunology" (8th Edition), Appleton & Lange, Norwalk, CT (1994); Mishell and Shiigi (Eds.), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., Ed. (1984); "Nucleic Acid Hybridization" Hames, B. D., and Higgins S. J., Eds. (1985); "Transcription and Translation" Hames, B. D., and Higgins S. J., Eds. (1984); "Animal Cell Culture" Freshney, R. I., Ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### DESIGN OF NON-TOXIC PERMANENT SELECTION IN CHO CELLS

### General background on the genes used for permanent selection

***The reduced folate carrier (RFC) -*** Folic acid and reduced folates are hydrophilic vitamins, essential for the biosynthesis of purines, thymidylate and certain amino acids in mammalian cells (Figure 1 and Stockstad, 1990). To support DNA synthesis, mammalian cells, unlike prokaryotes and plants, must rely on folate uptake from exogenous sources since they are unable to synthesize their own folate vitamins (Matherly and Goldman, 2003). Leucovorin (5-formyl-tetrahydrofolate) and related reduced folates including 5-methyl-tetrahydrofolate are precursors of one-carbon donors in the *de novo* biosynthesis of purines and pyrimidines. The primary cellular transport system for the uptake of reduced folates and folic acid antagonists (*i.e.* antifolates; see Figure 2) such as methotrexate (MTX;) is the human reduced folate carrier (hRFC; Figure 1, Zhao and Goldman, 2003). Figures 3a-b depict the chemical structure of folic acid and MTX.

The hRFC is an integral plasma membrane protein containing 591 amino acids (predicted molecular weight of ~65 kDa), with 12 transmembrane domains (TMDs), a short N-terminus and a long C-terminus both of which reside in the cytoplasm (Ferguson and Flintoff, 1999) (Figure 4). It contains a single N-linked glycosylation site in the first extracellular loop (L1) and undergoes heavy glycosylation which renders it a broadly migrating protein with a molecular mass of ~85 kDa (Matherly et al., 1992; Freisheim et al., 1992). RFC is a classic transporter that functions as a bidirectional anion exchanger (Goldman, 1971; Henderson and Zevely, 1981), taking up folate cofactors and exporting various organic anions (Henderson and Zevely, 1981) including thiamine pyrophosphate (Zhao et al., 2001a). RFC is a member of the major facilitator superfamily (MFS), a large group of carriers that transport diverse organic and inorganic compounds in both prokaryotes and eukaryotes (Pao et al., 1998). The central physiological role of the mammalian RFC has been recently confirmed in targeted disruption studies of the murine RFC locus. Zhao et al., (Zhao et al., 2001 b) recently demonstrated that homozygous disruption of the murine RFC gene is lethal at the embryonic stage and that maternal folic acid supplementation can rescue the embryonic lethality and results in early neonatal failure of hematopoietic organs.

Antifolates exert their cytotoxic activity by inhibiting specific enzymes in the folic acid metabolic pathway (Figure 2). For example, MTX, which enters the cell via the RFC, is a potent inhibitor of Di-Hydro Folate Reductase (DHFR), the first and key enzyme in folate metabolism (Figure 1). Exposure of cells to MTX results in folate-deficiency and failure to synthesize purines and deoxythymidylate. As a result, DNA synthesis is inhibited and the cells undergo apoptosis and cell death. Previous studies conducted by the present inventors demonstrated that exposure to MTX may lead to RFC inactivation which results in stable MTX-transport deficient phenotypes. Inactivating mutations in the hRFC coding region abolish the RFC transport activity and results in antifolate drug resistance (Jansen et al., 1998; Drori et al., 2000a; Rothem et al., 2002). In addition, silencing of the RFC promoter (primarily due to loss of expression and/or function of several transcription factors) caused loss of RFC gene expression and resulted in impaired MTX transport (Rothem et al., 2003; Rothem et al., 2004 a, b). In addition, although rare, hRFC promoter methylation has also been documented as a mechanism of hRFC gene silencing (Worm et al., 2001).

***Tire Galactokinase (GalK1) gene* -** D-galactose is an hexose derived from the hydrolysis of the disaccharide lactose (a component that is abundant in milk). D-galactose is converted to glucose-6-phosphate in a three-step enzymatic conversion (Figure 5 and Novelli and Reichardt, 2000). In the first step, galactokinase (GalK1; EC 2.7.1.6) in the presence of ATP converts D-galactose to D-galactose-1-phosphate; in the second step D-galactose-1-phosphate undergoes epimerization by UDP-Glucose-α-D-galactose-1-phosphate uridylyltransferase in the presence of UDP-glucose to D-glucose-1-phosphate; in the last step, D-glucose-1-phosphate is converted by phosphoglucomutase to D-glucose-6-phosphate, which can then be used in glycolysis. Recent studies demonstrated that lack of fully functional GalK1 is one cause of the inherited disease galactosemia, the main clinical manifestation of which is early onset cataracts (Novelli and Reichardt, 2000; Timson and Reece, 2003).

Galactokinase-deficient hamster cells (Schumperli et al., 1982) fail to grow in medium containing D-galactose as the sole hexose source and require a medium containing D-glucose for growth.

***Experimental design of permanent selection using the RFC gene -*** The first step of the RFC permanent selection approach requires establishment of RFC deficient CHO cells. After the cells are proven to lack the ability to transport folates, they are transfected with an expression vector carrying a reporter gene (e.g., EGFP) linked to the human RFC gene in a bi-cistronic configuration (see flowchart in Figure 6). Transfected cells are grown in G418 under selective folate concentrations (reduced leucovorin as the sole folate source) to select for RFC expressing cells. To allow transfected cells to internalize sufficient amounts of reduced folates and to survive under the folate deprivation conditions, the expression level of hRFC should be high. Once a stable transfected cell pool is established, leucovorin concentrations are gradually reduced and expression of GFP is followed upon removal of G418 to evaluate stability of expression in the absence of toxic selection. It is expected that under these conditions the expression of the GFP reporter gene should be stably maintained over time.

***Experimental design of permanent selection using the GalK1 gene -*** Galactokinase deficient cells (ATCC, CRL-1657) are transfected with an expression vector carrying a reporter gene (e.g., EGFP) linked to the human GalK1 gene (Stambolian et al., 1995) in a bi-cistronic configuration (see flowchart in Figure 7). Transfected cells are grown in G418 under selective galactose concentrations (5 mM D-galactose as the sole hexose source) to select for GalK1 expressing cells. Once a stable transfected cell pool is established D-galactose concentrations are gradually reduced and expression of GFP is then followed upon removal of G418 to evaluate stability of expression in the absence of toxic selection. It is expected that under these conditions the expression of the GFP reporter gene should be stably maintained over time.

### EXAMPLE 2

### ESTABLISHMENT OF PERMANENTLY SELECTED RFC EXPRESSING CELLS

***Isolation of MTX transport-deficient cells -*** The isolation of Chinese hamster ovary (CHO) AA8 cell mutants lacking RFC transporter activity has been previously described (Assaraf and Schimke, 1987). Wild type CHO AA8 cells were obtained from Dr. Cyntia Hoy originally from the lab of Dr. Larry H. Thompson: Lawrence Livemore National Laboratory, CA. Briefly, CHO AA8 cells (5 x 10⁵ cells/10 cm petri dish, in the presence of 20 ml medium) were grown as a monolayer in growth medium (αMEM, containing 2.3 µM folic acid) supplemented with 10 % fetal bovine serum, 2 mM glutamine and antibiotics. Cells were exposed to 75 nM or 150 nM MTX (Sigma) which correspond to -5- and 10-fold respectively of the 50 % lethal dose (LD50). MTX selection was carried out in the growth medium supplemented with 10 % dialyzed fetal calf serum (dFCS, GIBCO).

RFC- deficient CHO-S cells (Invitrogen, growing in suspension in chemically defined medium, CD-CHO, Invitrogen) were isolated by stepwise selection in gradually increasing concentrations of MTX. Cells were subjected to an initial concentration of 10 nM MTX and CHO-S RFC deficient cells were isolated in 150 nM MTX by gradually increasing the MTX concentration from 10 nM, through 40 nM, 120 nM and 150 nM in the medium over a period of~ five months.

***Construction of hRFC EGFP vectors -*** The hRFC cDNA was previously cloned in pcDNA3.1 (phRFC1), sequenced and the capacity of transport activity was confirmed (Drori et al., 2000b). The hRFC cDNA (SEQ ID NO:5) was excised from the pcDNA3.1 vector using the *Xho*I*-Bam*HI restriction enzymes from the phRFC1 mammalian expression vector and directionally cloned upstream of the IRES element in the pIRES-2-EGFP vector (Clontech) at the *Bgl*II*-Sal*I sites.

The hRFC-HA construct containing the human influenza virus hemagglutinin (HA) epitope tag (YPYDVPDYA) at the C-terminus of the hRFC was constructed as previously described (Liu and Matherly, 2002; Ferguson and Flintoff, 1999). The small HA epitope tag at the C-terminal end of the hRFC does not interfere with the folate/MTX transport activity of the RFC and enables the detection of the over-expressed hRFC-HA gene using commercial monoclonal antibodies. Noteworthy, such antibodies do not detect the endogenous inactivated hRFC gene that may possibly undergo some rare reversion and re-expression. The hRFC-HA construct was directionally cloned upstream of the IRES element in the pIRES-2-EGFP vector (Clontech) at the *Bgl II-Sal I* sites.

***Construction of the hGALK1-EGFP vector -*** The human GALK1 gene was cloned by PCR from a bacterial expression vector (pET21d, Novagen) obtained from Dr. Richard J. Reece, School of Biological Sciences, University of Manchester, UK (Timson and Reece, EJB, 2003); Dr. Reece obtained this GalK1 cDNA from the IMAGE consortium (clone ID: 3501788).

To amplify the full-coding region of the human GalK1 gene RT-PCR was performed using the following PCR primers: Forward; 5'-GAT CCG CTC GAG CCG CCA TGG CTG CTT TGA GAC AGC C-3' (SEQ ID NO:1) and Reverse; 5'-CCG GAA TTC ACA AGC ACA GCA CCT TGG C-3' (SEQ ID NO:2). The underlined hexanucleotide sequences encode a *Xho*I site and an *Eco*RI site, respectively. This GalK1 PCR product (SEQ ID NO:6) was resolved by agarose electrophoresis, purified using a gel extraction kit (Qiagen), digested with *Xho*I and *Eco*RI*,* and directionally cloned into pIRES2-EGFP vector (Clontech). The hGalK1-EGFP vector was used to transform competent Top1 bacteria (obtained from Prof. Dan Cassel, Department of Biology, Technion, Haifa 32000, Israel). Of the ~ 40 clones picked, 95 % contained a GalK1 insert as evidenced by both GalK1-specific PCR, as well as by *Xho*I-*Eco*RI excision of the GalKl insert. Sequencing of the positive transformants confirmed the presence of GalK1 gene.

### Establishment of stable transfectants

***hRFC-EGFP -*** Exponentially growing monolayer C5R0.15 cells (2 x 10⁷ cells) were detached by a standard trypsinization protocol whereas CHO-S cells growing in suspension were harvested by centrifugation. Cells were then transfected by electroporation (1000 microfarads, 234 V, Rothem et al., 2003) using 10 µg of the EGFP-based bicistronic expression vectors encoding the hRFC or hRFC-HA genes. Following transfection, cells were transferred to folic acid-free RPMI-1640 medium supplemented with 10 % dialyzed FCS, containing 2 nM leucovorin as the sole folate source and were grown at 37 °C for 48 hours. Cells were then transferred to RPMI-1640 medium lacking folic acid and supplemented with 10 % dialyzed fetal calf serum containing either 450 µg/ml or 600 µg/ml G-418 (Calbiochem-Novabiochem, San Diego, CA) and 2 nM leucovorin as the sole folate source. Following 5 weeks in the presence of 600 µg/ml G-418 + 2 nM LCV, the cells were subjected to a more stringent selection and transferred in parallel to 1, 0.5, and 0.25 nM leucovorin in the presence of a constant concentration of 600 µg/ml G-418. Cells were grown under these selective conditions for at least one month at which time cells were further grown in the absence or presence of G-418.

***hGALK1-EGFP -*** Exponentially growing GalK1-deficient monolayer cells (2 x 10⁷) were detached by a standard trypsinization protocol. Cells were then transfected by electroporation (1000 microfarads, 234 V) (Rothem et al., 2003) using 10 µg of the EGFP-based bicistronic expression vector encoding the GalK1 gene. Following transfection, cells were transferred to RPMI-1640 medium lacking glucose, supplemented with 10 % dialyzed fetal calf serum and containing either 5 mM or 2 mM D-galactose and grown at 37 °C for 24 hours. Cells were then transferred to RPMI-1640 medium lacking glucose, supplemented with 10 % dialyzed fetal calf serum and containing either 300, 450 or 600 µg/ml G-418 with either 5 mM or 2 mM D-galactose (6 independent pools). Following three days, cultures growing in 600 µg/ml G-418 + 5 mM D-galactose were split and transferred to a more stringent D-galactose and/or G-418 selection, as follows: 1 mM D-Galactose + 600 µg/ml G-418; 0.5 mM D-Galactose + 300 µg/ml G-418; 0.5 mM D-Galactose along + 750 µg/ml G-418. Following 40 days in the stringent conditions, cultures growing at 0.5 mM D-Galactose and 750 µg/ml G-418 were further placed in medium containing 0.25 mM or 0.1 mM D-Galactose in the presence or absence of 1 mg/ml G-418. These cultures grew for at least 5 weeks prior to analysis.

***Cell growth assays -*** In order to determine the doubling time of the host cells and their transfected derivatives, 2 x 10⁵ cells were seeded in 30 mm Petri dishes in the appropriate growth medium (5 ml growth medium/dish): RPMI 1640 +10 % FCS for the host cells, and RPMI 1640 lacking folic acid supplemented with 10 % dialyzed FCS, each in its respective selective media, for the transfectants. Cells were incubated in the growth medium for 4 days at 37 °C, during which the viable cell numbers were determined daily using trypan blue exclusion. The doubling time was calculated from the growth curves (cell numbers versus incubation time).

***Cell growth inhibition by antifolates** -* For antifolate cell growth inhibition, cells (10⁴/well) were seeded in 24-well plates in the respective growth medium (AA8 and derivatives in RPMI + 10 % FCS, and CHO-S cells in CD-CHO) containing various concentrations of MTX or the lipophilic antifolates, trimeterxate (Dr. David Fry, Warner-Lambert, Parke-Davis, Detroit, Michigen) and incubated for 3 days at 37 °C. Thereafter, cells were detached by trypsinization and the number of viable cells was determined using trypan blue exclusion. The 50 % inhibitory concentration (IC₅₀) is defined as the drug concentration at which cell growth is inhibited by 50 % relative to untreated controls.

***F-MTX staining and competition with hydrophilic and lipophilic antifolates*** - Cells were seeded in 60-mm Petri dish and incubated for 8 hours at 37 °C in growth medium containing 2 µM fluorescein-MTX (F-MTX). Cells were then washed with PBS and subjected to competition with MTX and its lipophilic analogue TMQ. Following 3 hours of incubation, cells were detached by trypsinization, suspended in PBS containing 1 % fetal calf serum and the residual green fluorescence per cell was determined using a flow cytometer (FACSCalibur, Becton-Dickinson) at an excitation of 488 nm and emission of 525 nm. Autofluorescence of unstained cells was routinely recorded.

***[³H]-MTX transport -*** In order to determine the ability of the antifolate-resistant clones to take up antifolates the influx rates of [³H]-MTX were measured and compared to the control CHO AA8 cells. Cells (2 x 10⁷) from the mid-log phase of growth were washed three times in transport buffer consisting of HEPES-buffered saline solution (HBSS, Rothem et al., 2002) and incubated for 3 minutes at 37 °C in the presence of HBSS (1 ml suspensions) containing 2 µM [³H]-MTX. Transport controls contained a 500-fold excess (1 mM) of unlabeled MTX. The transport of [³H]-MTX was stopped by the addition of 10 ml of ice-cold HBSS. The cell suspension was then centrifuged for 5 minutes at 4 °C using a centrifugation speed of 500 x g, following whhich the cell pellet was washed twice with 10 ml of ice-cold transport buffer. The final cell pellet was suspended in water and processed for scintillation counting.

***Isolation of MTX transport-deficient CHO AA8 cells -*** Chinese hamster ovary (CHO) AA8 cell mutants lacking RFC transporter activity were isolated by exposing wild type AA8 cells to 75 nM or 150 nM MTX concentrations, corresponding to ~5- and 10-fold (respectively) the 50 % lethal dose (LD₅₀) (Figures 11a-b). MTX selection was carried out in the growth medium supplemented with 10 % dialyzed fetal calf serum (dFCS, GIBCO). Following 18 days of exposure to MTX, 7 independent clones were picked (colony size ~80-300 cells) using sterile cloning rings. Of the seven independent CHO AA8 colonies that were initially picked (deriving from 75 and 150 nM MTX-resistant cells), only 2 clones (C4 R0.15 and C5 R0.15) both derived from selection in the presence of 150 nM MTX showed stable growth in MTX-selective medium. The remaining 5 clones displayed either unstable growth in selective medium and/or exhibited a polyploid phenotype and were therefore discarded.

***Isolation of RFC-deficient CHO-S cells -*** CHO-S cells growing in suspension in chemically defined medium were isolated by stepwise selection in gradually increasing concentrations of MTX up to 150 nM MTX (10, 40, 120, and 150 nM) over a period of ~ five months. Cells which were resistant to 150 nM MTX were designated CHO-SR0.15.

***RFC transport activity of MTX-resistant cell lines -*** To confirm that the MTX-resistant cell lines (CHO-S and CHO AA8) are impaired in their MTX transport activity, a flow cytometric assay of F-MTX staining followed by competition with hydrophilic (MTX) and lipophilic antifolates (TMQ) was employed. As is shown in Figure 8, CHO-S R0.15 cells displayed a bright F-MTX staining which was ~2-fold higher than that of CHO-S cells, suggesting an increased DHFR expression in the CHO-S R0.15 cells. As expected however, following incubation of cells in the presence of 100 nM MTX, the CHO-S cells completely lost their F-MTX staining (Figure 8). In contrast, incubation of CHO-S R0.15 cells with 100 nM MTX did not result in any loss of cellular F-MTX staining. Moreover, 1000 nM MTX were required for a complete displacement of F-MTX in the CHO-S R0.15 cells. On the other hand, incubation of either cell types with only 10 nM of the lipid-soluble antifolate TMQ that enters cells by diffusion, resulted in a complete loss of F-MTX fluorescence (Figure 8). Similarly, incubation of CHO AA8 cells with 30 nM MTX resulted in a complete loss of F-MTX fluorescence (Figure 9a). On the other hand, 3 µM of MTX were required to completely displace F-MTX fluorescence from the CHO AA8 C4R0.15 and CHO AA8 C5R0.15 cells (Figures 9b and c, respectively).

***[³H]-MTX transport assay -*** To provide direct evidence that the MTX-resistant cell lines of the present invention are indeed defective in MTX transport, the [³H]-MTX transport assay was employed. Initial rates of MTX uptake were determined using 2 µM [³H]-MTX over 3 minutes of transport. CHO-S cell lines, which were isolated in the early steps of the MTX stepwise selection in the presence of 40 and 120 nM MTX respectively, lost 92.5 % and 96.4 % of control CHO-S transport activity (Figure 11a). Similarly, the CHO AA8 C4 R0.15 and C5 R0.15 cell lines displayed 90-91 % loss of MTX uptake, relative to the control AA8 cells (Figure 11b). Hence, the very poor transport of MTX in these MTX-resistant cell lines provides direct evidence that RFC transport activity has been largely lost in these cells.

Cells which were confirmed to be RFC-deficient using both the [³H]-MTX transport assay and the Fluorescein-MTX staining assay were used as hosts for the transfection of the hRFC.

Altogether, these results demonstrate that significantly higher amounts of MTX are required to displace the intracellular F-MTX (which is present in a high-affinity complex with intracellular DHFR) within the RFC-deficient cells (*i.e.,* CHO-S R150, CHO AA8 C4R0.15 and Cur0.15 cells). These results therefore suggest that the CHO-S R150, CHO AA8 C4R0.15 and C5R0.15 cells are defective in MTX uptake.

***TMQ hypersensitivity of MTX-resistant cell lines -*** Cells that are impaired in RFC transport activity, typically display a phenotype of hypersensitivity to the lipid-soluble antifolates, trimetrexate (TMQ) and piritrexim (PTX) as they have a marked shrinkage in their intracellular folate pools (Rothem et al., 2002). To further characterize the sensitivity of the RFC-deficient cells to antifolates, the CHO-S R0.15, C4R0.15 and C5R0.15 cells were subjected to TMQ cytotoxicity experiments. As is shown in Figure 10, while in CHO-S cells 50 % inhibition of cell growth (i.e., IC₅₀) was achieved in the presence of approximately 40 nM of TMQ, in CHO-S R0.15 cells the IC₅₀ was 10 nM of TMQ (Figure 10). These results indicate the loss of RFC transport activity is associated with decreased intracellular folate pool.

Moreover, the MTX-resistant CHO AA8 C4R0.15 and C5R0.15 cells were >3-fold and >5-fold hypersensitive to PTX and TMQ, respectively, when compared to their CHO AA8 counterpart (IC₅₀ values ∼ 8 nM and 6 nM, respectively, data not shown).

These results provide further evidence to the loss of RFC transport activity thereby resulting in a marked diminishment in the intracellular folate pools and a consequent marked hypersensitivity to lipophilic antifolates.

### EXAMPLE 3

### PERMANENTLY SELECTED RFC - EXPRESSING CELLS MAINTAIN HIGH EXPRESSION LEVELS IN THE ABSENCE OF CYTOTOXIC DR UGS (G418)

***Extraction of membrane proteins from cultured cells -*** Exponentially growing cells were detached by trypsinization, washed three times with PBS and harvested by centrifugation. Cells (1 x 10⁶ - 3 x 10⁶) were then incubated in a lysis buffer containing 50 mM Tris-HCl at pH 7.5, 50 mM 2-mercaptoethanol, 0.5 % Triton X-100, and a complete^{™} mini mixture of protease inhibitors (Roche) containing 10 µg/ml phenylmethyl sulfonyl fluoride, 60 µg/ml aprotinin, 5 µg/ml leupeptin, 10 µg/ml pepstatin, 1 mM EGTA (pH 8), and 1 mM EDTA (pH 8) (Assaraf and Borgnia, 1994). Following 1 hour of incubation on ice, the protein extract was centrifuged and aliquots of the supernatant were stored at -80 °C until analysis. Protein content was determined using the Bio-Rad protein assay according to Bradford.

***Western blot analysis -*** RFC-HA expression was studied by Western blot analysis using anti-HA antibodies. Briefly, microsomal proteins were resolved by electrophoresis on 10 % polyacrylamide gels containing SDS and electroblotted onto nitrocellulose nylon membrane (Schleicher & Schuell). The blots were blocked for 1 hour at room temperature in TBS buffer (150 mM NaCl and 0.5 % Tween 20 and 10 mM Tris/Cl at pH 8.0) containing 1 % skim milk, following which they were incubated with 1:1000 dilutions of the H12 monoclonal antibody (Covance Inc., Princeton, NJ) specific to the HA epitope. Blots were then rinsed in the same buffer for 10 minutes in room temperature and were further incubated for 1 hour at room temperature with a secondary horseradish peroxidase (HRP) - conjugated goat anti-mouse antibody (IgG; 1:40,000 dilution, Jackson Immunoresearch Labs, West Grove, PA). Following three 10-min washes in TBS at room temperature, enhanced chemiluminescence detection was performed according to the manufacturer's instructions (Biological Industries, Beth Haemek, Israel). Protein content was determined using the Bradford protein assay (Bio-Rad).

***Immunofluorescence staining -*** Cells (10⁴) were seeded in 24-well plates (1 ml medium/well) on sterile glass coverslips and incubated for 3 days at 37 °C. For immunostaining, the growth medium was removed and monolayer cells were washed twice with PBS and fixed for 10 minutes using 4 % formaldehyde in PBS. Following fixation, the coverslips were washed twice with PBS, incubated for 20 minutes in a solution of 80 % methanol in double distilled water, washed twice with PBS, blocked for 1 hour at room temperature in PBS containing 1 % skim milk and reacted with an anti-HA monoclonal antibody H12 (Covance Inc., Princeton, NJ; 1:100 dilution). Following anti-HA incubation the coverslips were washed XXX times (XXX minutes each) in the presence of XXX (PBS?), and were further incubated with a secondary FITC-conjugated rabbit anti-mouse IgG (Sigma; 1:100). Following incubation with the secondary antibody the coverslips were washed twice with PBS and cell nuclei were stained for 60 minutes at room temperature with DAPI (Sigma; catalogue # D-9564) at a final concentration of 0.5 µg/ml. Following four washes with PBS (each with 2 ml), the coverslips were mounted onto glass slides using fluoromount-GTM (Southern Biotech; Birmingham, Alabama Catalog # 0100-01). The slides were then examined using a BioRad MRC1024 confocal microscope.

***Flow cytometric analysis of EGFP expression -*** Cells cultured in 25-mm tissue culture flasks were washed with PBS, detached by trypsinization and suspended in PBS + 1 % FCS at 10⁶ cells/ml. Cells were then analyzed for green fluorescence per cell using a flow cytometer (FACSCalibur, Becton- Dickinson) at an excitation of 488 nm and emission of 525 nm. Autofluorescence of unstained cells was routinely recorded.

***Cell growth assays and inhibifion by antifolates -*** as described in Example 2, hereinabove.

***Effect of leucovorin selection on reporter gene expression -*** The effect of decreasing leucovorin concentrations on the expression of the EGFP reporter gene. Flow cytometric analysis of the C5 R0.15 RFC-HA transfectants grown in G-418 (600 µg/ml) in the presence of decreasing concentrations of leucovorin (0.25-2 nM) revealed a 100-fold increase in EGFP fluorescence as compared to non-transfected control cells (Figure 12). Similarly, hRFC CHO-S R0.15, which were grown in the presence of 600 µg/ml G-418 and 2 nM or 10 nM leucovorin, displayed a ∼10-fold increase in EGFP fluorescence following transfection with the bicistronic vector encoding RFC or RFC-HA as selectable markers and EGFP as the second cistron (Figure 13). These results indicate the effectiveness of the leucovorin selection and suggest that the upstream genes, RFC or RFC-HA are expressed at high levels in these transfected cells.

***Confirmation of hRFC expression by Western Blot-*** The expression of RFC-HA in the RFC-HA transfected cells was further confirmed by Western blot analysis using monoclonal antibodies to the HA epitope. As is shown in Figure 14, CHO AA8 C5 R0.15 transfected cells growing in a selective medium containing 2 nM leucovorin and 600 µg/ml G-418 strongly expressed the hRFC-HA protein.

***Effect of hRFC expression on growth inhibition -*** To confirm that the transfected hRFC-HA cells exhibited a functional folate transporter, an MTX growth inhibition assay was carried out. RFC deficient cells overexpressing a functional hRFC receptor are expected to be hypersensitive to MTX as compared to their parental hosts. In these experiments the parental CHO AA8 C5 cells were found to have an MTX IC₅₀ value of ∼3 µM whereas the hRFC and hRFC-HA CHO AA8 C5R0.15 transfectants displayed an MTX IC₅₀ value of 27-29 nM, two orders of magnitude lower as compared to the parental cells (Figure 15). Hence, the hRFC and hRFC-HA transfected cells, expressed 100-fold more functional hRFC and hRFC-HA transporters as compared to the parental C5R0.15 cells.

***Immunofluorescence detection of RFC -*** In order to ascertain that the hRFC-HA was properly targeted to the plasma membrane, the transfected cells were subjected to immuofluorescent staining using an anti-HA antibody followed by confocal microscopy analysis. As is shown in Figures 16a-g, the green fluorescence corresponding to hRFC-HA overexpression was confined to the plasma membrane of the CHO AA8 C5R0.15 transfectants, whereas no staining was observed in the non-transfected cells (compare Figure 16f to Figure 16c). Counterstaining with the DAPI dye accurately localized the borders of the nuclei (Figures 16b and d).

***Growth of RFC transfected cell lines -*** The growth characteristics of the RFC deficient host cells and their transfectants were examined. CHO AA8 C5 R0.15 cells growing in a growth medium containing 2.3 µM folic acid exhibited a doubling time of -24 hours (Figure 17). Doubling times were also determined for the hRFC-HA transfectants which grew in folic acid-free medium containing 1-2 nM leucovorin in the presence of 600 µg/ml G-418 or after 3 weeks of G-418 removal. Under these various selective growth conditions the doubling times were ∼28 hours, similar to the doubling time of the CHO AA8 C5 R0.15 host cell line. In comparison, the parental AA8 cell line exhibited a doubling time of -15 hours (Figure 17). The longer doubling time of RFC deficient cells is a well-established phenomenon that is due to the marked shrinkage in the intracellular folate pools (Rothem et al., 2002).

These results indicate that the hRFC-HA transfectants retain a relatively long doubling times even in the presence of severe selective growth conditions

***Stability of expression under permanent selection conditions with RFC -*** To study the stability of the overexpressed reporter gene, flow cytometric analysis of EGFP was performed using various transfectants growing in the continuous presence of 1-2 nM leucovorin in the presence or absence of G-418. As is shown in Figures 18a-b, EGFP expression was stably maintained at nanomolar leucovorin concentrations for 2 months in the absence of G-418. In addition, hRFC-HA transfectants growing for two months under permanent selection conditions in medium containing only 1 nM leucovorin in the absence of G-418 appeared to have higher EGFP fluorescence than transfectants growing for only 3 weeks in G-418-free medium. These results clearly establish that a high level EGFP expression is retained in the hRFC-HA transfectants under permanent selection conditions.

### EXAMPLE 4

### PERMANENTLY SELECTED GALK- EXPRESSING CELLS MAINTAIN HIGH EXPRESSION LEVELS IN THE PRESENCE OF LOW D-GALACTOSE CONCENTRATIONS

### Materials and Experimental Methods - as in Examples 2 and 3, hereinabove.

***Transfection of galactokinase deficient hamster cells -*** Chinese Hamster lung cells deficient in galactokinase (GalK) were purchased from ATCC (CRL-1657). While these cells displayed normal growth in D-glucose-containing RPMI-1640 medium, they failed to grow in RPMI-1640 medium (Biological Industries, Beth-Haemek, Israel) containing as high as 10 mM D-galactose as the sole hexose source (data not shown).

***Stability of expression under galactose deprivation -*** In order to evaluate stability of expression of the reporter gene under permanent selection conditions with the GalK1 gene, and the effect of gradual deprivation of D-galactose, the levels of expression of the EGFP reporter gene were determined in the presence or absence of G-418. Flow cytometry analysis of the GalK1 transfectants grown for three weeks in 1 mg/ml G-418 and 0.25-0.5 mM D-galactose as the sole hexose source revealed two equally distributed sub-populations, which showed ∼5-fold and 100-fold EGFP overexpression, relative to non-transfected GalK-deficient cells (Figure 19a). After two months of growth in medium containing 0.25-0.5 mM D-galactose as the sole hexose source, the GalK1 transfectants became more homogenous with a dominant population stably expressing 100-fold EGFP regardless the presence of G-418 (1 mg/ml) in the D-galactose-containing medium (Figure 19b). These results suggest that the primary driving force of EGFP expression in these GalK1 transfectants is the gradual deprivation of D-galactose. Hence the reporter gene in this study (EGFP) was shown to be stably expressed for at least two months in the absence of toxic selection, under permanent selection conditions with the GalK1 gene. Since high levels GalK1 must be expressed in order to allow for cell growth at sub-millimolar concentrations of D-galactose, it is highly likely that the upstream GalK1 gene is overexpressed at levels which are comparable or higher to those of EGFP.

***Growth of hGalK1 transfected cell lines -*** The doubling times of the galactokinase-deficient host cells as well as their various GalK1 transfectants were determined (Figure 20). Parental hamster galactokinase-deficient cells displayed a doubling time of approximately ∼24 hours. The GalK1 transfectants growing in medium containing both 0.25 mM D-galactose and 1 mg/ml G-418 or D-galactose alone were found to have doubling times of 24-28 hours. However, as with hRFC-HA trasfectants, there was a significant increase in the lag time in the various GalK1 transfectants. These results demonstrate that the various GalK1 transfectants retain a relatively good doubling time even in the presence of severe selective growth conditions of as high as 1 mg/ml G418 and as low as 0.25 mM D-galactose.

To test the feasibility of employing selectable genes as permanent, non-toxic selection agents for the expression of a gene-of interest in a bioreactor, two novel selectable genes were employed: the reduced folate carrier (RFC) and the galactokinase (GalK) genes. Expression vectors containing the RFC or GalK1 genes along with a reporter gene (EGFP) were constructed and RFC- or Galk-deficient cells were stably transfected with the respective expression vectors.

This feasibility study confirmed that the two genes tested (the reduced folate carrier and the galactokinase) can be used for the purpose of permanent selection and that growth of cells in their respective selective media (in the absence of folates or in the presence of galactose as the sole hexose source) allows to maintain the stable expression of the reporter gene (EGFP) in the absence of toxic selection for at least two months.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

### REFERENCES

### (Additional references are cited in the text)

1. Assaraf, Y.G. and Schimke, R.T. (1987) Identification of methotrexate transport deficiency in mammalian cells using fluoresceinated methotrexate and flow cytometry. Proc. Natl. Acad. Sci. USA 84, 7154-7158.
2. Assaraf, Y.G. and Borgnia, M.J. (1994) Probing the interaction of the multidrug resistance phenotype with the polypeptide ionophore gramicidin D via functional channel formation. Eur. J. Biochem. 212, 813-824.
3. Cao, W. and Matherly L.H. (2003) Characterization of a cysteine-less human reduced folate carrier: localization of a substrate-binding domain by cysteine-scanning mutagenesis and cysteine accessibility methods. Biochem J.; 374 (Pt 1), 27-36
4. Drori, S., Jansen, G., Mauritz, R., Peters, G., and Assaraf, Y.G. (2000a) Clustering of mutations in the first transmembrane domain of the human reduced folate carrier in GW1843U89-resistant leukemia cells with impaired antifolate transport and augmented folate uptake. J. Biol. Chem. 275, 30855-30863.
5. Drori, S., Sprecher, H., Shemer, G., Jansen, G., Goldman, I.D. and Assaraf, Y.G. (2000b) Characterization of a human alternatively spliced truncated reduced folate carrier (RFC) increasing folate accumulation in parental leukemia cells. Eur. J. Biochem. 267, 1-14.
6. Ferguson, P.L., and Flintoff, W.F. (1999) Topological and functional analysis of the human reduced folate carrier by hemagglutinin epitope insertion. J. Biol. Chem. 274, 16269-16278
7. Freisheim, J., Ratnam, M., McAlinden, T.P., Prasad, K.M.R., Williams, F.E., Westerhof, G.R., Schornagel, J.H. and Jansen, G. (1992) Molecular events in membrane transport of methotrexate in human CCRF-CEM leukemia cells. Adv. Enzyme Regul. 32, 17-31.
8. Goldman, I.D. (1971) The characteristics of the membrane transport of amethopterin and the naturally occurring folates. Ann. NY Acad. Sci. 186, 400-422
9. Henderson, G.B., and Zevely, E.M. (1981) Anion exchange mechanism for transport of methotrexate in L1210 cells. Biochem. Biophys. Res. Commun. 99, 163-169
10. Jansen, G., Mauritz, R., Drori, S., Sprecher, H., Kathmann, I., Bunni, M., Priest, D.G., Noordhuis, P., Schornagel, J.H., Pinedo, H.M., Peters, G.J. and Assaraf, Y.G. (1998) A structurally altered human reduced folate carrier with increased folic acid transport mediates a novel mechanism of antifolate resistance. J. Biol. Chem. 273, 30189-30198.
11. Liu, X.Y., and Matherly, L.H. (2002) Analysis of membrane topology of the human reduced folate carrier protein by HA epitope insertion and scanning glycosylation insertion mutagenesis. Bichim. Biophys. Acta 1564, 332-342.
12. Matherly, L.H., Angeles, S.M., and Czajkowski, C.A. (1992) Characterization of transport-mediated methotrexate resistance in human tumor cells with antibodies to membrane carrier for methotrexate and tetrahydrofolate cofactors. J. Biol. Chem. 267, 23253-23260
13. Matherly, L.H., and Goldman, I.D. (2003) Membrane transport of folates. Vitam. Horm. 66, 403-456.
14. Novelli, G., Reichardt, J.K. (2000) Molecular basis of human galactose metabolism: past, present and future. Mol. Genet. Metab. 71, 62-65.
15. Pao, S.S., Paulsen, I.T., and Saier, M.H., Jr. (1998) Major facilitator superfamily. Microbiol. Mol. Rev. 62, 1-34.
16. Rothem, L., Ifergan, I., Kaufman, Y., Priest, D.G., Jansen, G., and Assaraf YG (2002) Resistance to multiple novel antifolates is mediated via defective drug transport resulting from clustered mutations in the reduced folate carrier gene in human leukaemia cell lines. Biochem. J. 367, 741-750.
17. Rothem, L., Aronheim, A., and Assaraf, Y.G. (2003) Alterations in the expression of transcription factors and the reduced folate carrier as a novel mechanism of antifolate resistance in human leukemia cells. J. Biol. Chem. 278, 8935-8941.
18. Rothem, L., Stark, M., Kaufman, Y., Mayo, L., and Assaraf, Y.G. (2004a) Reduced folate carrier gene silencing in multiple antifolate-resistant tumor cell lines is due to a simultaneous loss of function of multiple transcription factors but not promoter methylation. J. Biol. Chem. 279, 374-384.
19. Rothem, L., Stark, M., and Assaraf, Y.G. (2004b) Impaired CREB-1 phosphorylation in antifolate-resistant cell lines with down-regulation of the reduced folate carrier gene. Mol. Pharmacol., in press.
20. Schumperli, D., Howard, B.H., and Rosenberg, M. (1982) Efficient expression of Escherichia coli galactokinase gene in mammalian cells. Proc. Natl. Acad. Sci. USA 79, 257-261.
21. Stambolian, D., Ai, Y., Sidjanin, D., Nesburn, K., Sathe, G., Rosenberg, M., and Bergsma, D.J. (1995) Cloning of the galactokinase cDNA and identification of mutations in two families with cataracts. Nat. Genetics 10: 307-312.
22. Stockstad, E.L.R. Historical perspective on key advances in the biochemistry and physiology of folates. In: (Piccianno,M.F., Stockstad, E.L.R., Gregory, J.F. eds) Folic Acid Metabolism in Health and Disease. Wiley-Liss, New York, 1990, pp.1-21.
23. Timson, D.J., and Reece, R.J. (2003) Functional analysis of diseases-causing mutations in human galactokinase. Eur. J. Biochem. 270, 1764-1774.
24. Worm, J., Kirkin, A.F., Dzhandzhugazyan, K.N., Guldberg, P. (2001) Methylation-dependent silencing of the reduced folate carrier gene in inherently methotrexate-resistant human breast cancer cells. J. Biol. Chem. 26, 39990-40000.
25. Zhao, R., Gao, F., Wang, Y., Diaz, G.A., Gelb, B.D., and Goldman, I.D. (2001 a) Impact of the reduced folate carrier on the accumulation of active thiamin metabolites in murine leukemia cells. J. Biol. Chem. 276, 1114-1118.
26. Zhao, R., Russell, R.G., Wang, Y., Liu, L., Gao, F., Kneitz, B., Edelmann,W., and Goldman, I.D. (2001b) Rescue of embryonic lethality in reduced folate carrier-deficient mice by maternal folic acid supplementation reveals early neonatal failure of hematopoietic organs. J. Biol. Chem. 276, 10224-10228.
27. Zhao, R. and Goldman, I.D. (2003) Resistance to antifolates. Oncogene 22, 7431-7457.

## Claims

1. A method of selecting for positively-transformed cells comprising:
(a) providing eukaryotic cells lacking activity of an endogenous cell viability protein;
(b) transforming said eukaryotic cells with a nucleic acid construct encoding said cell viability protein or at least a functional portion thereof as well as further encoding a protein-of-interest; and
(c) culturing said eukaryotic cells transformed with said nucleic acid construct under conditions such that cell viability is dependent upon normal activity of said cell viability protein thereby selecting for positively-transformed cells,
wherein the cell viability protein is galactokinase 1, wherein the eukaryotic cell is a mammalian cell, wherein a nucleic acid coding for the said cell viability protein and a nucleic acid coding for said protein-of-interest are in bi-cistronic configuration with the nucleic acid sequence encoding the protein-of-interest being downstream the nucleic acid sequence encoding the cell viability protein, and wherein said culturing is effected in the presence of D-Galactose in a concentration of 0.25 mM to 0.5 mM.

2. The method of claim 1, wherein said protein-of-interest is selected from the group consisting of a growth factor, an hormone, a cytokine, an extracellular protein, a cell adhesion protein, and a cell signaling protein.

3. The method of claim 2, wherein said growth factor is selected from the group consisting of Epidermal Growth Factor, transforming growth factor-beta, fibroblast growth factor-acidic, fibroblast growth factor-basic, erythropoietin, thrombopoietin, hepatocyte growth factor, insulin-like growth factor-I, insulin-like growth factor-II, Interferon-gamma, and platelet-derived growth factor.

4. The method of claim 2, wherein said hormone is selected from the group consisting of prolactin, parathyroid hormone, Gastrin, leptin, growth hormone, insulin and CG-ß.

5. The method of claim 2, wherein said cytokine is selected from the group consisting of SDF-Iα, IL-7, IL-10, IL-20 and IL-19.

6. The method of claim 2, wherein said extracellular protein is selected from the group consisting of fibrinogen, Collagen, fibronectin, vimentin, microtubule- associated protein Ib, Neurite outgrowth factor (NOF), bacterial cellulose (BC), and laminin.

7. The method of claim 2, wherein said cell adhesion protein is selected from the group consisting of cell adhesion proteins include integrin, intercellular adhesion molecule (ICAM) 1, N-CAM, cadherin, tenascin, gicerin, and nerve injury induced protein 2 (ninjurin2).

8. The method of claim 2, wherein said cell signaling protein is selected from the group consisting of p38 mitogen-activated protein kinase, nuclear factor kappaB, Raf kinase inhibitor protein (RKIP), Raf-1, MEK, Protein kinase C (PKC), phosphoinositide-3-kinase gamma, receptor tyrosine kinases, heterotrimeric G- proteins, Caveolin-3, and 14-3-3 proteins.

9. The method of claim 1, wherein said nucleic acid construct includes a nucleic acid sequence as set forth in SEQ ID NO:3.

10. The method of claim 1, wherein a concentration of said D-Galactose is 0.25 mM.

11. A cytotoxic-free eukaryotic cell culture comprising cells genetically modified to express a cell viability protein and a protein-of-interest under culturing conditions such that cell viability is dependent upon normal activity of said cell viability protein, wherein the cell viability protein is galactokinase 1, wherein the eukaryotic cell is a mammalian cell and wherein a nucleic acid coding for the said cell viability protein and a nucleic acid coding for said protein-of-interest are in bi-cistronic configuration with the nucleic acid sequence encoding the protein-of-interest being downstream the nucleic acid sequence encoding the cell viability protein, and wherein said culturing is effected in the presence of D-Galactose in a concentration of 0.25 mM to 0.5 mM.

12. The cell culture of claim 11, wherein said protein-of-interest is selected from the group consisting of a growth factor, an hormone, a cytokine, an extracellular protein, a cell adhesion protein, and a cell signaling protein.

13. The cell culture of claim 12, wherein said growth factor is selected from the group consisting of Epidermal Growth Factor, transforming growth factor- beta, fibroblast growth factor-acidic, fibroblast growth factor-basic, erythropoietin, thrombopoietin, hepatocyte growth factor, insulin-like growth factor-I, insulin-like growth factor-II, Interferon-gamma, and platelet-derived growth factor.

14. The cell culture of claim 12, wherein said hormone is selected from the group consisting of prolactin, parathyroid hormone, Gastrin, leptin, growth hormone, insulin and CG-ß.

15. The cell culture of claim 12, wherein said cytokine is selected from the group consisting of SDF-I α, IL-7, IL-10, IL-20 and IL- 19.

16. The cell culture of claim 12, wherein said extracellular protein is selected from the group consisting of fibrinogen, Collagen, fibronectin, vimentin, microtubule-associated protein Ib, Neurite outgrowth factor (NOF), bacterial cellulose (BC), and laminin.

17. The cell culture of claim 12, wherein said cell adhesion protein is selected from the group consisting of integrin, intercellular adhesion molecule (ICAM) 1, N-CAM, cadherin, tenascin, gicerin, and nerve injury induced protein 2 (ninjurin2).

18. The cell culture of claim 12, wherein said cell signaling protein is selected from the group consisting of p38 mitogen-activated protein kinase, nuclear factor kappaB, Raf kinase inhibitor protein (RKIP), Raf-1, MEK, Protein kinase C (PKC), phosphoinositide-3-kinase gamma, receptor tyrosine kinases, heterotrimeric G-proteins, Caveolin-3, and 14-3-3 proteins.

19. The cell culture of claim 13, wherein a concentration of said D- Galactose is 0.25 mM.

20. A method of producing a protein-of-interest, comprising:
(a) transforming eukaryotic cells lacking activity of an endogenous cell viability protein with a nucleic acid construct encoding the protein-of-interest and said cell viability protein or at least a functional portion thereof;
(b) culturing said eukaryotic cells transformed with said nucleic acid construct under conditions such that cell viability is dependent upon normal activity of said cell viability protein thereby selecting for positively-transformed eukaryotic cells; and
(c) purifying the protein-of-interest from said positively-transformed eukaryotic cells or a culture medium thereof,
wherein the cell viability protein is galactokinase 1, wherein the eukaryotic cell is a mammalian cell and wherein a nucleic acid coding for the said cell viability protein and a nucleic acid coding for said protein-of-interest are in bi-cistronic configuration with the nucleic acid sequence encoding the protein-of-interest being downstream the nucleic acid sequence encoding the cell viability protein, and wherein said culturing is effected in the presence of D-Galactose in a concentration of 0.25 mM to 0.5 mM.

21. The method of claim 20, wherein said protein-of-interest is selected from the group consisting of a growth factor, an hormone, a cytokine, an extracellular protein, a cell adhesion protein, and a cell signaling protein.

22. The method of claim 21, wherein said growth factor is selected from the group consisting of Epidermal Growth Factor, transforming growth factor-beta, fibroblast growth factor-acidic, fibroblast growth factor-basic, erythropoietin, thrombopoietin, hepatocyte growth factor, insulin-like growth factor-I, insulin-like growth factor-II, Interferon-gamma, and platelet-derived growth factor.

23. The method of claim 22, wherein said hormone is selected from the group consisting of prolactin, parathyroid hormone, Gastrin, leptin, growth hormone, insulin and CG-ß.

24. The method of claim 22, wherein said cytokine is selected from the group consisting of SDF-I α, IL-7, IL-10, IL-20 and IL- 19.

25. The method of claim 22, wherein said extracellular protein is selected from the group consisting of fibrinogen, Collagen, fibronectin, vimentin, microtubule- associated protein Ib, Neurite outgrowth factor (NOF), bacterial cellulose (BC), and laminin.

26. The method of claim 22, wherein said cell adhesion protein is selected from the group consisting of integrin, intercellular adhesion molecule (ICAM) 1, N-CAM, cadherin, tenascin, gicerin, and nerve injury induced protein 2 (ninjurin2).

27. The method of claim 22, wherein said cell signaling protein is selected from the group consisting of p38 mitogen-activated protein kinase, nuclear factor kappaB, Raf kinase inhibitor protein (RKIP), Raf-1, MEK, Protein kinase C (PKC), phosphoinositide-3-kinase gamma, receptor tyrosine kinases, heterotrimeric G- proteins, Caveolin-3, and 14-3-3 proteins.

28. The method of claim 22, wherein said nucleic acid construct includes a nucleic acid sequence as set forth in SEQ ID NO:3.

29. The method of claim 21, wherein a concentration of said D-Galactose is 0.25 mM.

## Patentansprüche

1. Verfahren zum Selektieren von positiv transformierten Zellen, umfassend:
a) Bereitstellen eukaryotischer Zellen, denen die Aktivität eines endogenen Zellviabilitätsproteins fehlt;
b) Transformieren der eukaryotischen Zellen mit einem Nucleinsäurekonstrukt, welches das Zellviabilitätsprotein oder mindestens einen funktionellen Anteil davon kodiert sowie ferner ein Protein von Interesse kodiert, und
c) Kultivieren der mit dem Nucleinsäurekonstrukt transformierten eukaryotischen Zellen unter solchen Bedingungen, dass die Zellviabilität von normaler Aktivität des Zellviabilitätsproteins abhängt, wodurch auf positiv transformierte Zellen selektiert wird,
wobei es sich bei dem Zellviabilitätsprotein um Galactokinase 1 handelt, wobei es sich bei der eukaryotischen Zelle um eine Säugerzelle handelt, wobei eine Nucleinsäure, die für das Zellviabilitätsprotein kodiert, und eine Nucleinsäure, die für das Protein von Interesse kodiert, in bicistronischer Konfiguration vorliegen, wobei die Nucleinsäuresequenz, die das Protein von Interesse kodiert, stromabwärts von der Nucleinsäuresequenz liegt, die das Zellviabilitätsprotein kodiert, und wobei das Kultivieren in Anwesenheit von D-Galactose in einer Konzentration von 0,25 mM bis 0,5 mM bewirkt wird.

2. Verfahren nach Anspruch 1, wobei das Protein von Interesse aus der Gruppe ausgewählt ist, bestehend aus einem Wachstumsfaktor, einem Hormon, einem Zytokin, einem extrazellulären Protein, einem Zelladhäsionsprotein und einem Zell-Signalübertragungs-Protein.

3. Verfahren nach Anspruch 2, wobei der Wachstumsfaktor aus der Gruppe ausgewählt ist, bestehend aus epidermalem Wachstumsfaktor, transformierendem Wachstumsfaktor-beta, Fibroblasten-Wachstumsfaktor-sauer, Fibroblasten-Wachstumsfaktor-basisch, Erythropoietin, Thrombopoietin, Hepatozytenwachstumsfaktor, insulinähnlichem Wachstumsfaktor-I, insulinähnlichem Wachstumsfaktor-II, Interferon-gamma und Blutplättchen-Wachstumsfaktor.

4. Verfahren nach Anspruch 2, wobei das Hormon aus der Gruppe ausgewählt ist, bestehend aus Prolaktin, Parathormon, Gastrin, Leptin, Wachstumshormon, Insulin und CG-β.

5. Verfahren nach Anspruch 2, wobei das Zytokin aus der Gruppe ausgewählt ist, bestehend aus SDF-I α, IL-7, IL-10, IL-20 und IL-19.

6. Verfahren nach Anspruch 2, wobei das extrazelluläre Protein aus der Gruppe ausgewählt ist, bestehend aus Fibrinogen, Kollagen, Fibronektin, Vimentin, Mikrotubuli-assoziiertem Protein Ib, Neuriten-Auswuchs-Faktor (NOF), Bakteriencellulose (BC) und Laminin.

7. Verfahren nach Anspruch 2, wobei das Zelladhäsionsprotein aus der Gruppe ausgewählt ist, bestehend aus Zelladhäsionsproteinen, die Integrin, interzelluläres Adhäsionsmolekül (ICAM) 1, N-CAM, Cadherin, Tenascin, Gicerin und durch Nervenverletzung induziertes Protein 2 (Ninjurin2) einschließen.

8. Verfahren nach Anspruch 2, wobei das Zellsignalübertragungsprotein aus der Gruppe ausgewählt ist, bestehend aus p38-mitogenaktivierter Proteinkinase, nukleärem Faktor kappaB, Raf-Kinase-Inhibitorprotein (RKIP), Raf-1, MEK, Proteinkinase C (PKC), Phosphoinositid-3-Kinase gamma, Rezeptortyrosinkinasen, heterotrimeren G-Proteinen, Caveolin-3 und 14-3-3-Proteinen.

9. Verfahren nach Anspruch 1, wobei das Nucleinsäurekonstrukt eine in SEQ ID NO: 3 dargelegte Nucleinsäuresequenzenthält.

10. Verfahren nach Anspruch 1, wobei eine Konzentration der D-Galactose 0,25 mM beträgt.

11. Zytotoxin-freie eukaryotische Zellkultur, umfassend Zellen, die genetisch modifiziert sind, um ein Zellviabilitätsprotein und ein Protein von Interesse unter solchen Kultivierungsbedingungen zu exprimieren, dass Zellviabilität von normaler Aktivität des Zellviabilitätsproteins abhängt, wobei es sich bei dem Zellviabilitätsprotein um Galactokinase 1 handelt, wobei es sich bei der eukaryotischen Zelle um eine Säugerzelle handelt, und wobei eine Nucleinsäure, die für das Zellviabilitätsprotein kodiert, und eine Nucleinsäure, die für das Protein von Interesse kodiert, in bicistronischer Konfiguration vorliegen, wobei die Nucleinsäuresequenz, die das Protein von Interesse kodiert, stromabwärts von der Nucleinsäuresequenz liegt, die das Zellviabilitätsprotein kodiert, und wobei das Kultivieren in Anwesenheit von D-Galactose in einer Konzentration von 0,25 mM bis 0,5 mM bewirkt wird.

12. Zellkultur nach Anspruch 11, wobei das Protein von Interesse aus der Gruppe ausgewählt ist, bestehend aus einem Wachstumsfaktor, einem Hormon, einem Zytokin, einem extrazellulären Protein, einem Zelladhäsionsprotein und einem Zellsignalübertragungsprotein.

13. Zellkultur nach Anspruch 12, wobei der Wachstumsfaktor aus der Gruppe ausgewählt ist, bestehend aus epidermalem Wachstumsfaktor, transformierendem Wachstumsfaktor-beta, Fibroblasten-Wachstumsfaktor-sauer, Fibroblasten-Wachstumsfaktor-basisch, Erythropoietin, Thrombopoietin, Hepatozytenwachstumsfaktor, insulinähnlichem Wachstumsfaktor-I, insulinähnlichem Wachstumsfaktor-II, Interferon-gamma und Blutplättchen-Wachstumsfaktor.

14. Zellkultur nach Anspruch 12, wobei das Hormon aus der Gruppe ausgewählt ist, bestehend aus Prolaktin, Parathormon, Gastrin, Leptin, Wachstumshormon, Insulin und CG-β.

15. Zellkultur nach Anspruch 12, wobei das Zytokin aus der Gruppe ausgewählt ist, bestehend aus SDF-I α, IL-7, IL-10, IL-20 und IL-19.

16. Zellkultur nach Anspruch 12, wobei das extrazelluläre Protein aus der Gruppe ausgewählt ist, bestehend aus Fibrinogen, Kollagen, Fibronektin, Vimentin, Mikrotubuli-assoziiertem Protein Ib, Neuriten-Auswuchs-Faktor (NOF), Bakteriencellulose (BC) und Laminin.

17. Zellkultur nach Anspruch 12, wobei das Zelladhäsionsprotein aus der Gruppe ausgewählt ist, bestehend aus Integrin, interzellulärem Adhäsionsmolekül (ICAM) 1, N-CAM, Cadherin, Tenascin, Gicerin und durch Nervenverletzung induziertem Protein 2 (Ninjurin2).

18. Zellkultur nach Anspruch 12, wobei das Zellsignalübertragungsprotein aus der Gruppe ausgewählt ist, bestehend aus p38-mitogenaktivierter Proteinkinase, nukleärem Faktor kappaB, Raf-Kinase-Inhibitorprotein (RKIP), Raf-1, MEK, Proteinkinase C (PKC), Phosphoinositid-3-Kinase gamma, Rezeptortyrosinkinasen, heterotrimeren G-Proteinen, Caveolin-3 und 14-3-3-Proteinen.

19. Zellkultur nach Anspruch 13, wobei eine Konzentration der D-Galactose 0,25 mM beträgt.

20. Verfahren zum Herstellen eines Proteins von Interesse, umfassend:
a) Transformieren eukaryotischer Zellen, denen die Aktivität eines endogenen Zellviabilitätsproteins fehlt, mit einem Nucleinsäurekonstrukt, welches das Protein von Interesse und das Zellviabilitätsprotein oder mindestens einen funktionellen Anteil davon kodiert;
b) Kultivieren der mit dem Nucleinsäurekonstrukt transformierten eukaryotischen Zellen unter solchen Bedingungen, dass die Zellviabilität von normaler Aktivität des Zellviabilitätsproteins abhängt, wodurch auf positiv transformierte eukaryotische Zellen selektiert wird, und
c) Reinigen des Proteins von Interesse aus den positiv transformierten eukaryotischen Zellen oder einem Kulturmedium davon,
wobei es sich bei dem Zellviabilitätsprotein um Galactokinase 1 handelt, wobei es sich bei der eukaryotischen Zelle um eine Säugerzelle handelt, und wobei eine Nucleinsäure, die für das Zellviabilitätsprotein kodiert, und eine Nucleinsäure, die für das Protein von Interesse kodiert, in bicistronischer Konfiguration vorliegen, wobei die Nucleinsäuresequenz, die das Protein von Interesse kodiert, stromabwärts von der Nucleinsäuresequenz liegt, die das Zellviabilitätsprotein kodiert, und wobei das Züchten in Anwesenheit von D-Galactose in einer Konzentration von 0,25 mM bis 0,5 mM bewirkt wird.

21. Verfahren nach Anspruch 20, wobei das Protein von Interesse aus der Gruppe ausgewählt ist, bestehend aus einem Wachstumsfaktor, einem Hormon, einem Zytokin, einem extrazellulären Protein, einem Zelladhäsionsprotein und einem Zellsignalübertragungsprotein.

22. Verfahren nach Anspruch 21, wobei der Wachstumsfaktor aus der Gruppe ausgewählt ist, bestehend aus epidermalem Wachstumsfaktor, transformierendem Wachstumsfaktor-beta, Fibroblasten-Wachstumsfaktor-sauer, Fibroblasten-Wachstumsfaktor-basisch, Erythropoietin, Thrombopoietin, Hepatozytenwachstumsfaktor, insulinähnlichem Wachstumsfaktor-I, insulinähnlichem Wachstumsfaktor-II, Interferon-gamma und Blutplättchen-Wachstumsfaktor.

23. Verfahren nach Anspruch 22, wobei das Hormon aus der Gruppe ausgewählt ist, bestehend aus Prolaktin, Parathormon, Gastrin, Leptin, Wachstumshormon, Insulin und CG-β.

24. Verfahren nach Anspruch 22, wobei das Zytokin aus der Gruppe ausgewählt ist, bestehend aus SDF-I α, IL-7, IL-10, IL-20 und IL-19.

25. Verfahren nach Anspruch 22, wobei das extrazelluläre Protein aus der Gruppe ausgewählt ist, bestehend aus Fibrinogen, Kollagen, Fibronektin, Vimentin, Mikrotubuli-assoziiertem Protein Ib, Neuriten-Auswuchs-Faktor (NOF), Bakteriencellulose (BC) und Laminin.

26. Verfahren nach Anspruch 22, wobei das Zelladhäsionsprotein aus der Gruppe ausgewählt ist, bestehend aus Integrin, interzellulärem Adhäsionsmolekül (ICAM) 1, N-CAM, Cadherin, Tenascin, Gicerin und durch Nervenverletzung induziertem Protein 2 (Ninjurin2).

27. Verfahren nach Anspruch 22, wobei das Zellsignalübertragungsprotein aus der Gruppe ausgewählt ist, bestehend aus p38-mitogenaktivierter Proteinkinase, nukleärem Faktor kappaB, Raf-Kinase-Inhibitorprotein (RKIP), Raf-1, MEK, Proteinkinase C (PKC), Phosphoinositid-3-Kinase gamma, Rezeptortyrosinkinasen, heterotrimeren G-Proteinen, Caveolin-3 und 14-3-3-Proteinen.

28. Verfahren nach Anspruch 22, wobei das Nucleinsäurekonstrukt eine in SEQ ID NO: 3 dargelegte Nucleinsäuresequenz einschließt.

29. Verfahren nach Anspruch 21, wobei eine Konzentration der D-Galactose 0,25 mM beträgt.

## Revendications

1. Une méthode de sélection pour des cellules positivement transformées comprenant:
(a) la fourniture des cellules eucaryotes manquant d'activité de protéine endogène de viabilité cellulaire;
(b) la transformation desdites cellules eucaryotes avec un assemblage d'acides nucléiques codant ladite protéine de viabilité cellulaire ou au moins une portion fonctionnelle de celle-ci ainsi que codant une protéine d'intérêt ; et
(c) la culture desdites cellules eucaryotes transformées avec ledit assemblage d'acide nucléique dans des conditions telles que la viabilité cellulaire soit dépendante de l'activité normale de ladite protéine viabilité cellulaire en sélectionnant ainsi la viabilité des cellules transformées positivement,
dans laquelle la protéine de viabilité cellulaire est une galactokinase 1, dans laquelle la cellule eucaryote est une cellule de mammifère, dans laquelle un acide nucléique codant pour ladite protéine de viabilité cellulaire et un acide nucléique codant pour ladite protéine d'intérêt sont en configuration bi-cistronique avec la séquence d'acide nucléique codant pour la protéine d'intérêt étant en aval de la séquence d'acide nucléique codant pour la protéine de la viabilité cellulaire, et dans laquelle ladite culture est effectuée en présence de la D-Galactose à une concentration de 0.25 mM à 0.5 mM.

2. La méthode selon la revendication 1, dans laquelle ladite protéine d'intérêt est sélectionnée parmi le groupe constituant en un facteur de croissance, une hormone, une cytokine, une protéine extracellulaire, une protéine d'adhésion cellulaire et une protéine de signalisation cellulaire.

3. La méthode selon la revendication 2, dans laquelle ledit facteur de croissance est sélectionné parmi le groupe consistant en un facteur de croissance épidermique, un facteur de croissance transformant bêta, un facteur de croissance du fibroblaste acide, un facteur de croissance du fibroblaste de base, une érythropoïétine, une thrombopoïétine, un facteur de croissance d'hépatocyte, un facteur de croissance de type insuline I, un facteur de croissance de type insuline II, un interféron gamma, et un facteur de croissance dérivé des plaquettes.

4. La méthode selon la revendication 2, dans laquelle ladite hormone est sélectionnée parmi le groupe consistant en une prolactine, une hormone parathyroïde, une gastrine, une leptine, une hormone de croissance, de l'insuline, et CG-ß.

5. La méthode selon la revendication 2, dans laquelle ladite cytokine est sélectionnée parmi le groupe consistant en une protéine SDF-Iα, IL-7, IL-10, IL-20 et IL-19.

6. La méthode selon la revendication 2, dans laquelle ladite protéine extracellulaire est sélectionnée parmi le groupe consistant en du fibrinogène, du collagène, une fibronectine, une vimentine, une protéine Ib associé aux microtubules, un facteur d'excroissance du neurite (NOF), de la cellulose bactérienne (BC), et de la laminine.

7. La méthode selon la revendication 2, dans laquelle ladite protéine d'adhésion cellulaire est sélectionnée parmi le groupe consistant en des protéines d'adhésion cellulaire qui incluent une intégrine, une molécule d'adhésion intra-cellulaire (ICAM), N-CAM, une cadhérine, une tenascine, une gicerine, et une protéine induisant une lésion nerveuse 2 (ninjurin2).

8. La méthode selon la revendication 2, dans laquelle ladite protéine de signalisation cellulaire est sélectionnée parmi le groupe consistant en une protéine kinase p38 activée par des agents mitogènes, un facteur nucléaire kappaB, une protéine kinase Raf inhibitrice (RKIP), une protéine Raf-1, MEK, une protéine kinase C (PKC), une kinase 3 phosphoinositide gamma, un récepteur de la tyrosine kinase, une protéine G hétérotrimérique, une cavéoline 3, and des protéines 14-3-3.

9. La méthode selon la revendication 1, dans laquelle ledit assemblage d'acides nucléiques comprend une séquence d'acides nucléiques selon SEQ ID NO:3.

10. La méthode selon la revendication 1, dans laquelle la concentration de ladite D-galactose est de 0.25 mM.

11. Une culture de cellules eucaryotes libre cytotoxique comprenant des cellules génétiquement modifiées pour exprimer une protéine de la viabilité cellulaire et une protéine d'intérêt dans des conditions telles que la viabilité cellulaire dépende de l'activité normale de ladite protéine de viabilité cellulaire, dans laquelle la protéine de viabilité cellulaire est une galactokinase 1, dans laquelle la cellule eucaryote est une cellule de mammifère, et dans laquelle un acide nucléique codant pour ladite cellule protéine de viabilité et un acide nucléique codant pour ladite protéine d'intérêt sont en configuration bi-cistronique avec la séquence d'acide nucléique codant pour la protéine d'intérêt étant en aval de la séquence d'acide nucléique codant pour la protéine de viabilité cellulaire, et dans laquelle ladite culture est effectuée en présence de D-Galactose à une concentration de 0,25 mM à 0,5 mM.

12. La culture cellulaire selon la revendication 11, dans laquelle ladite protéine d'intérêt est sélectionnée parmi le groupe constituant en un facteur de croissance, une hormone, une cytokine, une protéine extracellulaire, une protéine d'adhésion cellulaire et une protéine de signalisation cellulaire.

13. La culture cellulaire selon la revendication 12, dans laquelle ledit facteur de croissance est sélectionné parmi le groupe consistant en un facteur de croissance épidermique, un facteur de croissance transformant bêta, un facteur de croissance du fibroblaste acide, un facteur de croissance du fibroblaste de base, une érythropoïétine, une thrombopoïétine, un facteur de croissance d'hépatocyte, un facteur de croissance de type insuline I, un facteur de croissance de type insuline II, un interféron gamma, et un facteur de croissance dérivé des plaquettes.

14. La culture cellulaire selon la revendication 12, dans laquelle ladite hormone est sélectionnée parmi le groupe consistant en une prolactine, une hormone parathyroïde, une gastrine, une leptine, une hormone de croissance, de l'insuline, et CG-ß.

15. La culture cellulaire selon la revendication 12, dans laquelle ladite cytokine est sélectionnée parmi le groupe consistant en une protéine SDF-I α, IL-7, IL-10, IL-20 et IL-19.

16. La culture cellulaire selon la revendication 12, dans laquelle ladite protéine extracellulaire est sélectionnée parmi le groupe consistant en un fibrinogène, du collagène, une fibronectine, une vimentine, une protéine Ib associée aux microtubules, un facteur d'excroissance du neurite (NOF), de la cellulose bactérienne (BC), et de la laminine.

17. La culture cellulaire selon la revendication 12, dans laquelle ladite protéine d'adhésion cellulaire est sélectionnée parmi le groupe consistant en des protéines d'adhésion cellulaire qui incluent une intégrine, une molécule d'adhésion intra-cellulaire (ICAM), N-CAM, une cadhérine, une tenascine, une gicerine, et une protéine induisant une lésion nerveuse 2 (ninjurin2).

18. La culture cellulaire selon la revendication 12, dans laquelle la protéine de signalisation cellulaire est sélectionnée parmi le groupe consistant en une protéine kinase p38 activée par des agents mitogènes, un facteur nucléaire kappaB, une protéine kinase Raf inhibitrice (RKIP), une protéine Raf-1, MEK, une protéine kinase C (PKC), une kinase 3 phosphoinositide gamma, un récepteur de la tyrosine kinase, une protéine G hétérotrimérique, une cavéoline 3, and des protéines 14-3-3.

19. La culture cellulaire selon la revendication 13, dans laquelle la concentration de ladite D-galactose est de 0.25 mM.

20. Une méthode produisant une protéine d'intérêt, comprenant:
(a) la transformation de cellules eucaryotes manquant d'activité de protéine endogène de viabilité cellulaire avec un assemblage d'acides nucléiques codant la protéine d'intérêt et ladite protéine de viabilité cellulaire ou au moins une portion fonctionnelle de celle-ci;
(b) la culture desdites cellules eucaryotes transformées avec ledit assemblage d'acides nucléiques dans des conditions telles que la viabilité des cellules dépende de l'activité normale de ladite protéine de viabilité cellulaire en sélectionnant ainsi la viabilité de cellules eucaryotes transformées positivement; et
(c) la purification de la protéine d'intérêt à partir desdites cellules eucaryotes positivement transformées ou un milieu de culture de celles-ci,
dans laquelle la protéine de viabilité cellulaire est une galactokinase 1, dans laquelle la cellule eucaryote est une cellule de mammifère, et dans laquelle un acide nucléique codant pour ladite protéine de viabilité cellulaire et un acide nucléique codant pour ladite protéine d'intérêt sont en configuration bi-cistronique avec la séquence d'acide nucléique codant pour la protéine d'intérêt étant en aval de la séquence d'acide nucléique codant pour la protéine de la viabilité cellulaire, et dans laquelle ladite culture est effectuée en présence de la D-Galactose à une concentration de 0.25 mM à 0.5 mM.

21. La méthode selon la revendication 20, dans laquelle ladite protéine d'intérêt est sélectionnées parmi le groupe constituant en un facteur de croissance, une hormone, une cytokine, une protéine extracellulaire, une protéine d'adhésion cellulaire et une protéine de signalisation cellulaire.

22. La méthode selon la revendication 21, dans laquelle ledit facteur de croissance est sélectionné parmi le groupe consistant en un facteur de croissance épidermique, un facteur de croissance transformant bêta, un facteur de croissance du fibroblaste acide, un facteur de croissance du fibroblaste de base, une érythropoïétine, une thrombopoïétine, un facteur de croissance d'hépatocyte, un facteur de croissance de type insuline I, un facteur de croissance de type insuline II, un interféron gamma, et un facteur de croissance dérivé des plaquettes.

23. La méthode selon la revendication 22, dans laquelle ladite hormone est sélectionnée parmi le groupe consistant en une prolactine, une hormone parathyroïde, une gastrine, une leptine, une hormone de croissance, de l'insuline, et CG-ß.

24. La méthode selon la revendication 22, dans laquelle ladite cytokine est sélectionnée parmi le groupe consistant en une protéine SDF-I α, IL-7, IL-10, IL-20 et IL- 19.

25. La méthode selon la revendication 22, dans laquelle ladite protéine extracellulaire est sélectionnées parmi le groupe consistant en un fibrinogène, du collagène, une fibronectine, une vimentine, une protéine Ib associé aux microtubules, un facteur d'excroissance du neurite (NOF), de la cellulose bactérienne (BC), et de la laminine.

26. La méthode selon la revendication 22, dans laquelle la protéine d'adhesion cellulaire est sélectionnée parmi le groupe consistant en une intégrine, une molecule d'adhésion intracellulaire (ICAM) 1, N-CAM, une cadhérine, une tenascine, une gicerine, et une protéine de lésion nerveuse induite 2 (ninjurin2).

27. La méthode selon la revendication 22, dans laquelle la protéine de signalisation cellulaire est sélectionnée parmi le groupe consistant en une protéine kinase p38 activée par des agents mitogènes, un facteur nucléaire kappaB, une protéine kinase Raf inhibitrice (RKIP), une protéine Raf-1, MEK, une protéine kinase C (PKC), une kinase 3 phosphoinositide gamma, un récepteur de la tyrosine kinase, une protéine G hétérotrimérique, une cavéoline 3, and des protéines 14-3-3.

28. La méthode selon la revendication 22, dans laquelle ledit assemblage d'acides nucléiques comprend une séquence d'acides nucléiques selon SEQ ID NO:3.

29. La méthode selon la revendication 21, dans laquelle la concentration de ladite D-Galactose est de 0.25 mM.
